# EUROPÄISCHE PATENTSCHRIFT

(11) **EP 0 481 405 B1**
(45) Veröffentlichungstag und Bekanntmachung des Hinweises auf die Patenterteilung: **11.06.1997**
(21) Anmeldenummer: 91117494.4
(22) Anmeldetag: 14.10.1991
(51) Int. Cl.: C07D 401/04, A01N 43/54

(54) **Pyrimidin-Derivate, Verfahren zu ihrer Herstellung sie enthaltende Mittel und ihre Verwendung als Fungizide**
Pyrimidin derivatives, process for their préparation, agents containing them and their use as fungicides
Dérivés de pyrimidine, procédé pour leur préparation, agents les contenant et leur utilisation comme fongicides

(30) Priorität: 17.10.1990 DE 4032878
(43) Veröffentlichungstag der Anmeldung: 22.04.1992
(73) Patentinhaber: Hoechst Schering AgrEvo GmbH, 13342 Berlin (DE)
(72) Erfinder: Vermehren, Jan, Dr., W-6238 Hofheim am Taunus (DE); Giencke, Wolfgang, Dr., W-6238 Hofheim am Taunus (DE); Braun, Peter, Dr., W-6500 Mainz (DE); Sachse, Burkhard, Dr., W-6233 Kelkheim(Taunus) (DE)

(56) Entgegenhaltungen:
- EP-A- 10 420
- EP-A- 106 515
- EP-A- 134 439
- EP-A- 278 610
- EP-A- 314 427
- EP-A- 0 270 362
- DE-A- 3 213 974
- US-A- 4 927 827

## Beschreibung

Die vorliegende Erfindung betrifft Pyrimidin Derivate, Verfahren zu ihrer Herstellung, sie enthaltende Mittel und ihre Verwendung als Fungizide.

Pyrimidin-Derivate sind bereits als wirksame Komponenten in fungiziden Mitteln bekannt (vgl. EP-A-270 362, EP-A-259 139, EP-A-234 104). Die Wirkung dieser Pyrimidin-Derivate ist jedoch, insbesondere bei niedrigen Aufwandmengen, nicht immer befriedigend.

Es wurden neue Pyrimidin-Derivate gefunden, die vorteilhafte Wirkungen bei der Bekämpfung eines breiten Spektrums phytopathogener Pilze, insbesondere bei niedrigen Dosierungen, aufweisen.

Gegenstand der vorliegenden Erfindung sind daher Verbindungen der Formel I 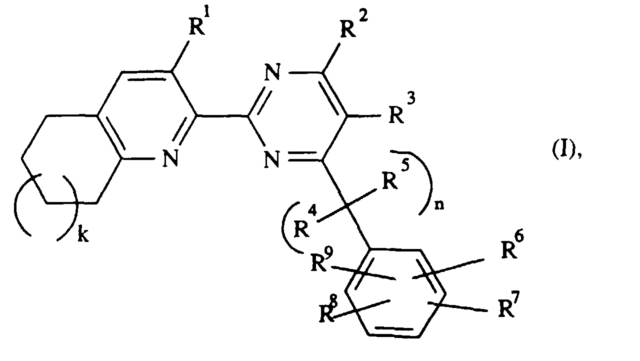 worin
- R¹ =: Wasserstoff, (C₁-C₆)Alkyl, (C₁-C₆)Alkoxy,Phenyl-(C₁-C₄)alkyl, wobei der Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
- R² =: Wasserstoff, Hydroxy, (C₂-C₄)Alkenyl-(C₁-C₄)alkyl, (C₂-C₄)Alkinyl-(C₁-C₄)alkyl, (C₁-C₆)Alkyl, (C₁-C₄)Alkoxy-(C₁-C₄)alkyl, (C₂-C₆)Alkenyl, (C₂-C₆)Alkinyl, (C₃-C₇)Cycloalkyl, (C₃-C₇)Cycloalkyl-(C₁-C₄)alkyl, wobei die beiden letztgenannten Reste im Cycloalkylteil bis zu dreifach durch (C₁ -C₄)Alkyl substituiert sein können, Phenyl,Phenoxy-(C₁-C₄)alkyl, Phenyl-(C₁-C₄)alkyl, wobei die drei letztgenannten Reste im Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, (C₁-C₁₂)Alkoxy, (C₂-C₆)Alkenyl-(C₁-C₄)alkoxy, (C₂-C₆)Alkinyl-(C₁-C₄)alkoxy, (C₃-C₇)Cycloalkoxy, (C₁-C₆)Alkoxy-(C₁-C₆)alkoxy, Hydroxy-(C₁-C₆)alkoxy, (C₁-C₆)Haloalkoxy, (C₁-C₆)Alkylthio, (C₂-C₆)Alkenyl-(C₁-C₄)alkylthio, (C₂-C₆)Alkinyl-(C₁-C₄)alkylthio, (C₃-C₇)Cycloalkylthio, Phenoxy, Phenylthio, Phenyl-(C₁-C₆)alkoxy, Phenyl-(C₁-C₆)alkylthio, Phenyl-(C₁-C₂)alkoxy-(C₁-C₄)alkoxy, wobei der Phenylring in den fünf letztgenannten Resten bis zu dreifach durch Halogen, (C₁-C₄)Alkyl, (C₁-C₄)Alkoxy, (C₁-C₄)Alkylthio, (C₁-C₄)Haloalkyl oder(C₁-C₄)Haloalkoxy sowie einfach durch Nitro oder Cyano substituiert sein kann,
- R³ =: Wasserstoff, (C₁-C₆)Alkyl, (C₁-C₄)Haloalkyl, (C₁-C₄)Alkoxy, (C₁-C₄)Alkylthio, Halogen, Phenyl, Phenyl-(C₁-C₄)alkyl, wobei der Phenylrest der drei letztgenannten Reste bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy sowie einfach durch Nitro oder Cyano substituiert sein kann,
- R⁴, R⁵ =: unabhängig voneinander oder Wasserstoff, (C₁-C₆)Alkyl, oder
R³ mit R⁴ oder R⁵ Bestandteil eines maximal ungesättigten 1-gliedrigen Ringes mit 1 = 3 bis 8,
- R⁶, R⁷, R⁸, R⁹ =: unabhängig voneinander Wasserstoff, Halogen, Nitro, Cyano, (C₁-C₆)Alkyl, (C₁-C₆)Alkoxy, (C₁-C₆)Alkylthio, (C₁-C₆)Haloalkyl, (C₁-C₆)Haloalkoxy, Aryl, Aryloxy, wobei die beiden letztgenannten Reste im Arylrest bis zu dreifach durch Halogen, (C₁-C₄)Alkyl, (C₁-C₄)Alkoxy, (C₁-C₄)Haloalkyl oder (C₁-C₄)Haloalkoxy substituiert sein können,
zwei der Reste R⁶ bis R⁹ gegebenenfalls Bestandteil eines ungesättigten oder gesättigten m-gliedrigen Ringes mit m= 5 oder 6,
- k =: 0, 1 oder 2,
- n =: 1 oder 2 und
- Halo =: ein- oder mehrfach durch Halogenatome substituiert bedeuten, sowie deren Säureadditionssalze.

Bevorzugt unter den Verbindungen der Formel I sind solche, worin
- R¹ =: Wasserstoff, (C₁-C₄)Alkyl, (C₁-C₆)Alkoxy,
- R² =: Wasserstoff, Hydroxy, (C₁-C₄)Alkyl, (C₂-C₄)Alkenyl-(C₁-C₂)alkyl, (C₂-C₄)Alkinyl-(C₁-C₂)alkyl, (C₁-C₄)Alkoxy-(C₁-C₄)alkyl, (C₂-C₄)Alkenyl, (C₂-C₄)Alkinyl, (C₃-C₆)Cycloalkyl, (C₃-C₆)Cycloalkyl-(C₁-C₂)alkyl, wobei die beiden letztgenannten Reste im Cycloalkylteil bis zu dreifach durch (C₁-C₄)Alkyl substituiert sein können, Phenyl, Phenoxy-(C₁-C₂)alkyl, Phenyl(C₁-C₂)alkyl, wobei die drei letztgenannten Reste im Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, (C₁-C₁₂)Alkoxy, (C₂-C₄)Alkenyl-(C₁-C₂)alkoxy, (C₂-C₄)Alkinyl-(C₁-C₂)alkoxy, (C₃-C₆)Cycloalkyloxy, (C₁-C₄)Alkoxy-(C₁-C₄)alkoxy, Hydroxy-(C₁-C₄)alkoxy, (C₁-C₆)Haloalkoxy, (C₁-C₆)Alkylthio, (C₂-C₆)Alkenyl-(C₁-C₂)alkylthio, (C₂-C₄)Alkinyl-(C₁-C₂)alkylthio, (C₃-C₆)Cycloalkylthio, Phenoxy, Phenylthio, Phenyl-(C₁-C₂)alkoxy, Phenyl-(C₁-C₂)alkylthio, Phenyl-(C₁-C₂)alkoxy-(C₁-C₄)alkoxy, wobei der Phenylring in den vier letztgenannten Resten bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
- R³ =: Wasserstoff, (C₁-C₄)Alkyl, (C₁-C₄)Haloakyl, (C₁-C₄)Alkoxy, (C₁-C₄)Alkylthio, Halogen, Phenyl, Phenyl-(C₁-C₂)alkyl,
- R⁴, R⁵ =: unabhängig voneinander Wasserstoff oder (C₁-C₂)Alkyl oder
R³ mit R⁴ oder R⁵ Bestandteil eines maximal ungesättigten 1-gliedrigen Ringes mit 1 = 5 oder 6,
- R⁶, R⁷, R⁸, R⁹ =: unabhängig voneinander Wasserstoff, Halogen, Nitro, Cyano, (C₁-C₄)Alkyl, (C₁-C₄)Alkoxy, (C₁-C₄)Haloalkyl, (C₁-C₄)Haloalkoxy, Phenyl, Phenyloxy, wobei die beiden letztgenannten Reste im Phenylrest bis zu dreifach durch Halogen, (C₁-C₄)Alkyl, (C₁-C₄)Alkoxy, (C₁-C₄)Haloalkyl oder (C₁-C₄)Haloalkoxy substituiert sein können, zwei der Reste R⁶ bis R⁹ gegebenenfalls Bestandteil eines ungesättigten oder gesättigten m-gliedrigen Ringes mit m = 5 oder 6,

- k =: 0, 1 oder 2,
- n =: 1 oder 2 und
- Halo =: ein- oder mehrfach durch Halogenatome substituiert bedeuten,
sowie deren Säureadditionssalze.

Dabei können die Alkyl-, Alkenyl-oder Alkinylreste sowohl gradkettig als auch verzweigt sein. Halogen bedeutet F, Cl, Br, J, bevorzugt F, Cl, Br. Die Vorsilbe "Halo" in der Bezeichnung eines Substituenten bedeutet hier und im Folgenden, daß dieser Substituent einfach oder mehrfach bei gleicher oder verschiedener Bedeutung auftreten kann. Die Vorsilbe "Halo" beinhaltet Fluor, Chlor, Brom oder Jod, insbesondere Fluor, Chlor oder Brom. Als Beispiele für Halogenalkane seien genannt: CF₃, CF₂CHF₂, CCl₃, CCl₂F, CF₂CF₂CF₃, CF₂CHFCF₃, CH₂CF₃ und (CF₂)₃CF₃.

Zur Herstellung der Säureadditionssalze der Verbindungen der Formel I kommen folgende Säuren in Frage:

Halogenwasserstoffsäuren wie Chlorwasserstoffsäure oder Bromwasserstoffsäure, ferner Phosphorsäure, Salpetersäure, Schwefelsäure, mono- oder bifunktionelle Carbonsäuren und Hydroxycarbonsäuren wie Essigsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionssalze der Verbindungen der Formel I können in einfacher Weise nach üblichen Salzbildungsmethoden, z.B. durch Lösen in einem organischen Lösemittel und Hinzufügen der Säure erhalten werden und in bekannter Weise, z.B. durch Abfiltrieren, isoliert und gegebenenfalls durch Waschen mit einem inerten Lösemittel gereinigt werden.

Gegenstand der Vorliegenden Erfindung ist auch ein Verfahren zur Herstellung der Verbindungen der Formel I.

Die neuen Pyrimidin-Derivate der Formel I können nach den folgenden Methoden hergestellt werden:
a) Pyrimidin-Derivate der Formel I mit R² = H werden entsprechend der folgenden Reaktionsgleichung durch reduktive Dehalogenierung von entsprechenden Halopyrimidinen der Formel I, in denen R² für Halogen (Cl, Br, J) steht und die restlichen Substituenten wie in Formel I definiert sind, erhalten. Die Dehalogenierung kann mit Wasserstoff in Gegenwart von Katalysatoren (z.B. Palladium/Kohle) in einem inerten Lösungsmittel z.B. Wasser, niederer Alkohol (wie Methanol und Ethanol), Ethylacetat oder Toluol oder Gemischen derselben durchgeführt werden. Vorteilhaft ist die Zugabe von Basen wie Alkali- oder Erdalkalihydroxide bzw. -carbonate. Die Reaktion wird vorzugsweise im Bereich von 15-60°C unter einem Wasserstoff-Druck von 1 bis 5 bar durchgeführt.
b) Pyrimidin-Derivate der Formel I, worin R² für einen mit dem Suffix "oxy" oder "thio" endenden, wie eingangs bei R² definierten organischen Rest steht, werden durch Reaktion von entsprechenden Halopyrimidinen der Formel I (R² = Halogen) mit einer Alkalimetallverbindung der Formel R²-Y (II), worin R² wie oben angegeben definiert ist und Y für ein Alkalimetall steht, erhalten. Beispiele für Alkalimetalle sind Natrium, Kalium und Lithium. Die Reaktion wird zwischen 0°C und 130°C innerhalb von 0,5 bis 72 Stunden durchgeführt. Die Alkalimetallverbindung R²-Y wird in Mengen von 1 bis 2 Moläquivalenten, bezogen auf 1 Äquivalent des Halopyrimidins (I) (R² = Halogen), eingesetzt. Die Reaktion wird in Gegenwart eines Lösungsmittels durchgeführt.
   Wird eine Alkalimetallverbindung R²-Y der Formel II eingesetzt, worin R² für (C₁-C₁₂)Alkoxy, (C₂-C₆)Alkenyl-(C₁-C₄)alkoxy, (C₂-C₆)Alkinyl-(C₁-C₄)alkoxy, (C₁-C₄)Haloalkoxy, (C₁-C₄)Alkoxy-(C₁-C₄)alkoxy, Phenyl-(C₁-C₂)alkoxy-(C₁-C₄)alkoxy oder Phenyl(C₁-C₂)alkoxy steht, wird zweckmäßigerweise der korrespondierende Alkohol R²-H oder ein Ether (z.B. Diethylether, Dioxan oder Tetrahydrofuran) oder eine Mischung derselben als Lösungsmittel benutzt. In den Fällen, in denen eine Alkalimetallverbindung R²-Y eingesetzt wird, worin R² für (C₁-C₆)Alkylthio, (C₁-C₄)Alkylthio-(C₁-C₄)alkylthio, Phenoxy, Phenylthio oder Phenyl-(C₁-C₂)alkylthio steht, wird ein Ether (z.B. Diethylether, Dioxan oder Tetrahydrofuran), ein Nitril (z.B. Acetonitril), ein aromatischer Kohlenwasserstoff (z.B. Toluol oder Xylol) oder eine Mischung derselben als Lösungsmittel verwendet.
c) Pyrimidin-Derivate der Formel I, worin R² für einen nicht mit dem Suffix "oxy" oder "thio" endenden, wie eingangs bei R² definierten organischen Rest steht, werden durch Umsetzung von entsprechenden Halopyrimidinen I (R² = Halogen) mit Grignardverbindungen R²-MgX (III), wobei R² wie oben angegeben definiert ist und X für Halogen (Cl, Br, J) steht, in Gegenwart eines Katalysators, beispielsweise 1,2-Bis-(diphenylphosphino)-ethan-nickel-(II)-chlorid oder 1,2-Bis-(diphenylphosphino)-propan-nickel-(II)-chlorid, erhalten (vgl. Chem. Pharm. Bull. Bd. 26, 2160 (1978) und Pure & Appl. Chem. Bd. 52, 669 (1980)). Die Reaktion wird zwischen 0°C und 80°C bzw. beim Siedepunkt des Lösungsmittels durchgeführt. Die Grignardverbindung R²-MgX der Formel III wird in Mengen von 1 bis 2,5 Moläquivalenten, bezogen auf 1 Äquivalent Halopyrimidin (I), eingesetzt. Als Lösungsmittel eignen sich Ether wie Diethylether, Dioxan, Tetrahydrofuran oder Dimethoxyethan.

Die Halopyrimidine der Formel I können durch Umsetzung der entsprechenden Hydroxypyrimidine I (R² = OH), worin die Reste R¹ und R³ bis R⁹ wie in der allgemeinen Formel I definiert sind, mit Halogenierungsreagenzien erhalten werden. Als Halogenierungsreagenz können z.B. Thionylchlorid, Phosgen, Phosphoroxychlorid, Phosphorpentachlorid, Phosphoroxybromid oder Phosphortribromid eingesetzt werden. Die Reaktionen können in einem Lösungsmittel, aber auch ohne Lösungsmittel durchgeführt werden. Das Halogenierungsreagenz wird in Mengen von 1 bis 4 Äquivalenten, bezogen auf 1 Äquivalent des Hydroxypyrimidins I (R² = OH), eingesetzt.

Die Reaktionen werden in einem Temperaturbereich von 25-160°C durchgeführt. Als Lösungsmittel werden vorzugsweise aromatische Kohlenwasserstoffe (z.B. Benzol oder Toluol, u.a.) oder halogenierte Kohlenwasserstoffe (z.B. Chlorbenzol, Dichlormethan oder 1,2-Dichlorethan) eingesetzt.

Die Hydroxypyrimidine der Formel I mit R² = OH sind neu und können nach literaturbekannten Verfahren (vgl. G.W.Miller, F.L.Rose, J.Soc.Chem. 1963, 5643) hergestellt werden.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytophathogener Pilze, wie z.B. Piricularia oryzae, Leptosphaeria nodorum, Pyrenophora teres, echte Mehltauarten, verschiedene Rostpilze und Botrytis cinerea, sowie die Oomycetenpilze Plasmopara viticola und Phytophthora infestans.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch Mittel, die die Verbindungen der Formel I neben geeigneten Formulierungshilfsmitteln enthalten. Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher in Frage: Spritzpulver (WP), emulgierbare Konzentrate (EC), wäßrige Dispersionen auf Öl- oder Wasserbasis (SC), Suspoemulsionen (SC), Stäubemittel (DP), Beizmittel, Granulate in Form von wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in:
Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:
Watkins, "Handbook of Insecticide Dust Diluents and Carrier", 2nd Ed., Darland Book, Caldwell N.J.; H.v. Olphen, "Introduction to Clay Colloid Chemistry, 2nd Ed., J. Wiley & Sons, N.Y.; Marschen, "Solvents Guide", 2nd Ed., Interscience, N.Y. 1950; McCutcheon's "Detergents and Emulsifiers Annual", MC Publ. Corp. Ridgewood N.J., Sisley and Wood, "Encyclopedia of Surface Active Agents", Chem. Publ. Co. Inc., N.Y. 1964; Schönfeldt, "Grenzflächenaktive Äthylenoxiaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'- disulfonsaures Natrium, dibutylnaphthalinsulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:
Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid- Sorbitanfettsäureester, Polyoxyethylensorbitan- Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% Bei Granulaten hängt der Wirkstoffgehalt zum Teil davon ab, ob die wirksame Verbindung flüssig oder fest vorliegt und welche Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken. Sie liegt zwischen 0,005 und 10,0 kg/ha Wirkstoff, vorzugsweise jedoch zwischen 0,01 und 5 kg/ha Wirkstoff.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z.B. folgende Produkte zu nennen:
Imazalil, Prochloraz, Fenapanil, SSF 105, Triflumizol, PP969 Flutriafol, BAY-MEB 6401, Propiconazol, Etaconazol, Tebuconazol, Diclobutrazol, Bitertanol,Triadimefon, Triadimenol, Fluotrimazol, Dimethomorph, Tridemorph, Dodemorph, Fenpropimorph, Falimorph, S-32165, Chlobenzthiazone, Parinol, Buthiobat, Fenpropidin, Triforine, Fenarimol, Nuarimol, Triarimol, Ethirimol, Dimethirimol, Bupirimate, Rabenzazole, Tricyc lazole, Fluobenzimine, Pyroxyfur, NK-483, PP-389, Pyroquilon Hymexazole, Fenitropan, UHF-8227, Cymoxanil, Dichlofunanid, Captafol, Captan, Folpet, Tolyfluanid, Chlorothalonil, Etridiazol, Iprodione, Procymidon, Vinclozol, Metomeclan, Myclozolin, Dichlozolinate, Fluorimide, Drazoxolan, Chinomethionate, Nitrothalisopropyl, Dithianon, Dinocap, Binapacryl, Fentinacetate, Fentinhydroxide, Carboxin, Oxycarboxin, Pyracarolid, Methfuroxam, Fenfura, Furmecyclos, Benodanil, Mebenil, Mepronil, Flutalanil, Fuberidazole, Thiabendazole, Carbendazim, Benomyl, Flusilazole, Metalaxyl, Pyrifenox, Furalaxyl, Methasulfocarb, Probenazole, Oxadixyl, Diniconazole, Cyprofuran, Fenpiclonil, Hexaconazole, Difluconazole, Iprobenfos, Edifenfos, Diethofencarb, Thiofante, Thiofanatemethyl, CGD-95340 F, IKF-1216, Mancozeb, Maneb, Zineb, Nabam, Thiram, Probineb, Prothiocarb, Propamocarb, Dodine, Guazatine, Dicloran, Quintozene, Chloroneb, Tecnazene, Biphenyl, Anilazine, 2-Phenylphenol, Kupferverbindungen wie Cu-oxychlorid, Oxine-Cu, Cu-oxide, Schwefel, Fosethylaluminium, Natrium-dodecylbenzolsulfonat, Natrium-dodecylsulfat, Natrium-C13/C15-alkoholethersulfonat, Natrium-cetostearylphosphatester, Dioctyl-natriumsulfosuccinat, Natrium-isopropylnaphthalinsulfonat, Natrium-methylenbisnaphthalinsulfonat, Cetyl-trimethyl-ammoniumchlorid,

Salze von langkettigen primären, sekundären oder tertiären Aminen, Alkyl-propylenamine, Lauryl-pyridinium-bromid, ethoxilierte quaternierte Fettamine, Alkyl-dimethyl- benzylammoniumchlorid und 1-Hydroxyethyl-2-alkyl-imidazolin.

Die oben genannten Kombinationspartner stellen bekannte Wirkstoffe dar, die zum großen Teil in CH.R. Worthing, U.S.B. Walker und The Pesticide Manual, 7. Aufl. (1983), British Crop Protection Council beschrieben sind.

Darüber hinaus können die erfindungsgemäßen Wirkstoffe in ihren handelsüblichen Formulierungen sowie in denaus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a..

Bevorzugte Mischungspartner sind:
1. aus der Gruppe der Phosphorsäureester Azinphos-ethyl, Azinphosmethyl, 1-(4-Chlorphenyl)-4-(O-ethyl, s-propyl)-phosphoryloxypyrazol (TIA-230), Chlorpyrifos, Coumaphos, Demeton, Demeton-S-methyl, Diazinon, Dichlorvos, Dimethoat, Ethoprophos, Etrimfos, Fenitrothion, Fenthion, Heptenophos, Parathion, Parathion-methyl, Phosalon, Pirimiphos-ethyl, Pirimiphos-methyl, Profenofos, Prothiofos, Sulprofos, Triazophos, Trichlorphon.
2. aus der Gruppe der Carbamate
   Aldicarb, Bendiocarb, BPMC (2-(1-Methylpropyl)- phenylmethylcarbamat), Butocarboxim, Butoxicarboxim, Carbaryl, Carbofuran, Carbosulfan, Cloethocarb, Isoprocarb, Methomyl, Oxamyl, Primicarb, Promecarb, Propoxur, Thiodicarb.
3. aus der Gruppe der Carbonsäureester
   Allethrin, Alphamethrin, Bioallethrin, Bioresmethrin, Cycloprothrin, Cyfluthrin, Cyhalothrin, Cypermethrin, Deltamethrin, 2,2-Dimethyl-3-(2-chlor-2-trifluormethylvinyl)-cyclopropancarbonsäure-(alpha-cyano-3-phenyl-2-methyl-benzyl)-ester (FMC 54800), Fenpropathrin, Fenfluthrin, Fenvalerat, Flucythrinate, Flumethrin, Fluvalinate, Permethin, Resmethrin, Tralomethrin.
4. aus der Gruppe der Formamidine
   Amitraz, Chlordimeform
5. aus der Gruppe der Zinnverbindungen
   Azocyclotin, Cyhexatin, Fenbutatinoxid
6. Sonstige
   Abamektin, Bacillus thuringiensis, Bensultap, Binapacyl, Bromopropylate, Buprofecin, Camphechlor, Cartap, Chlorbenzialate, Chlorfluazuron, 2-(4-Chlorphenyl)-4,5-diphenylthiophen (UBI-T 930), Chlofentezine, Cyclopropancarbonsäure(2-naphthylmethyl)-ester Ro 12-0470) Cyromacin, DDT, Dicofol, N-(3,5-Dichlor-4-(1,1,2,2-tetrafluoroethoxy)phenylamino)carbonyl)-2,6-difluorbenzamide (XRD 473), Diflubenzuron, N-(2,3-Dihydro-3-methyl-1,2-thiazol-2-ylidene)2,4- xylidine, Dinobuton, Dinocap, Endosulfan, Fenoxycarb. Fenthiocarb, Flubenzimine, Flufenoxuron, Gamma-HCH Hexythiazox, Hydramethylnon (AC 217 300) Ivermectin, 2-Nitromethyl-4,5-dihydro-6H-thiazin (SD 52618), 2-Nitromethyl-3,4-dihydrothiazol (SD 35651), 2-Nitromethylene-1,3-thiazinan-3-yl-carbamaldehyde (WL 108 477), Propargite, Teflubenzuron, Tetradifon, Tetrasul, Thicyclam, Triflumaron Kernpolyeder- und Granuloseviren.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 100 Gew.-% Wirkstoff, vorzugsweise von 0,001 bis 1 Gew.-% liegen. Die Anwendung geschieht in einer den Anwendungsformen angepaßten üblichen Weisen.

Nachfolgende Beispiele dienen zur Erläuterung der Erfindung.

### A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.- Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikro vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 AeO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### B. Chemische Beispiele

### Beispiel 1

### 4-(Methylnaphth-2-yl)-2-(5,6,7,8-tetrahydrochinolin-2-yl)-pyrimidin

Es wurde zu einer Suspension von 2,0 g (0,0052 mol) 4-Chlor-6-(methylnaphth-2-yl)-2-(5,6,7,8-tetrahydrochinolin-2-yl)-pyrimidin und 0,64 g (0,006 mol) Natriumcarbonat in 150 ml abs. Methanol unter Argon 50 mg Palladium 5%ig auf Kohle gegeben. In einer Wasserstoffatmosphäre wurde 16 Stunden intensiv bei 25°C gerührt. Der Feststoff wurde abfiltiert, das Filtrat eingeengt und der Rückstand flash-chromatographiert (Kieselgel/Ethylacetat). Es wurde 1,3 g (71%) eines weißen Feststoffes mit dem Schmelzpunkt 156°C bis 158°C erhalten.

### Beispiel 2

### 6-(2,4-Dichlorbenzyl)-4-ethoxy-2-(5,6,7,8-tetrahydrochinolin-2-yl)-pyrimidin

Zu der Lösung von 2,0 g (0,005 mol) 4-Chlor-6-(2,4-dichlorbenzyl)-2-(5,6,7,8-tetrahydrochinolin-2-yl)-pyrimidin in 50 ml abs. Ethanol wurden 1,95 g (0,006 mol) einer 21%igen Lösung von Natriumethylat in Ethanol getropft. Es wurde 16 Stunden bei 25°C gerührt und das Lösungsmittel im Vakuum abdestilliert. Der Rückstand wurde in Wasser gelöst, mit Dichlormethan dreimal extrahiert, die organische Phase getrocknet (MgSO₄) und eingeengt. Nach dem Trocknen im Vakuum wurden 1,7 g (85%) eines weißen Feststoffes vom Schmelzpunkt 98°C bis 100°C erhalten.

### Beispiel 3

### 4-Benzyl-6-methyl-2-(5,6,7,8-tetrahydrochinolin-2-yl)-pyrimidin

Zu der Suspension von 50 mg (0,00009 mol) 1,3-Bis-(diphenylphosphino)-propan-nickel-(II)-chlorid und 1,0 g (0,003 mol) 6-Benzyl-4-chlor-2-(5,6,7,8-tetrahydrochinolin-2-yl)-pyrimidin in 20 ml abs. THF wurden unter Argon bei 0°C 2 ml einer 3 molaren Lösung (0,006 mol) von Methylmagnesiumbromid in Diethylether unter intensiven Rühren zugetropft. Es wurde 8 Stunden unter Rückfluß erhitzt und anschließend 18 Stunden bei Raumtemperatur gerührt. Dann wurde auf verdünnte Salzsäure gegossen, 30 Minuten gerührt und mit Na₂CO₃ neutralisiert. Die organische Phase wurde getrocknet, eingeengt und der Rückstand flash-chromatographiert (Kieselgel, Ethylacetat). Es wurden 0,4 g (42%) eines gelben Öls erhalten.

Analog zu diesen Beispielen lassen sich die Verbindungen der Tabelle 1 herstellen.

Die Wasserstoff-Positionen der ¹H-NMR-Spektren werden wie nachfolgend aufgeführt belegt:

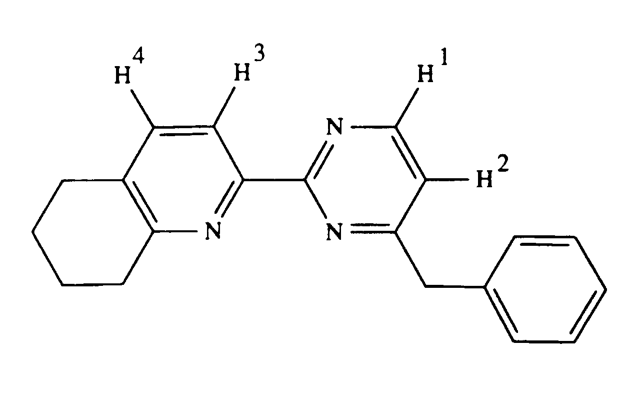

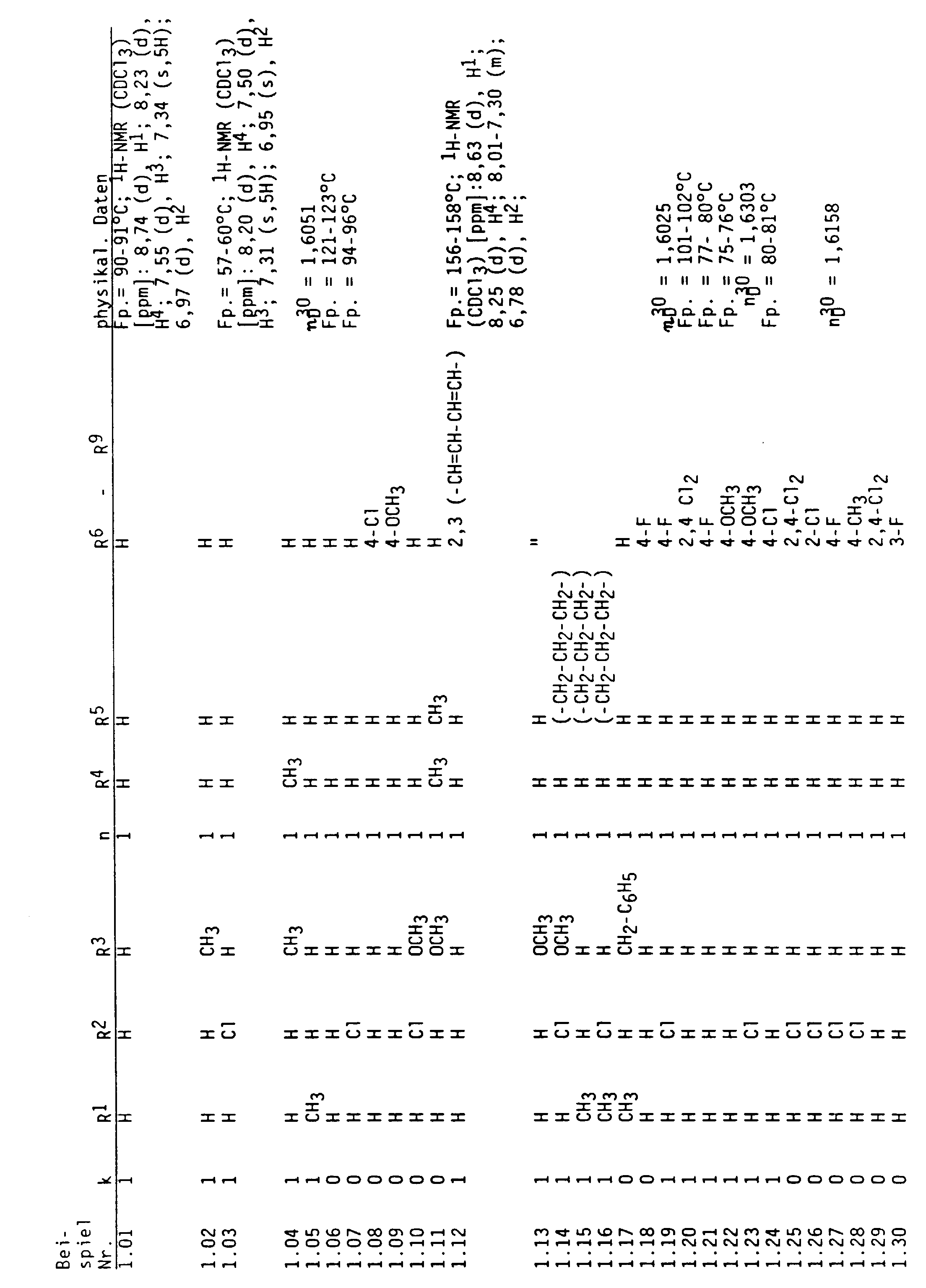

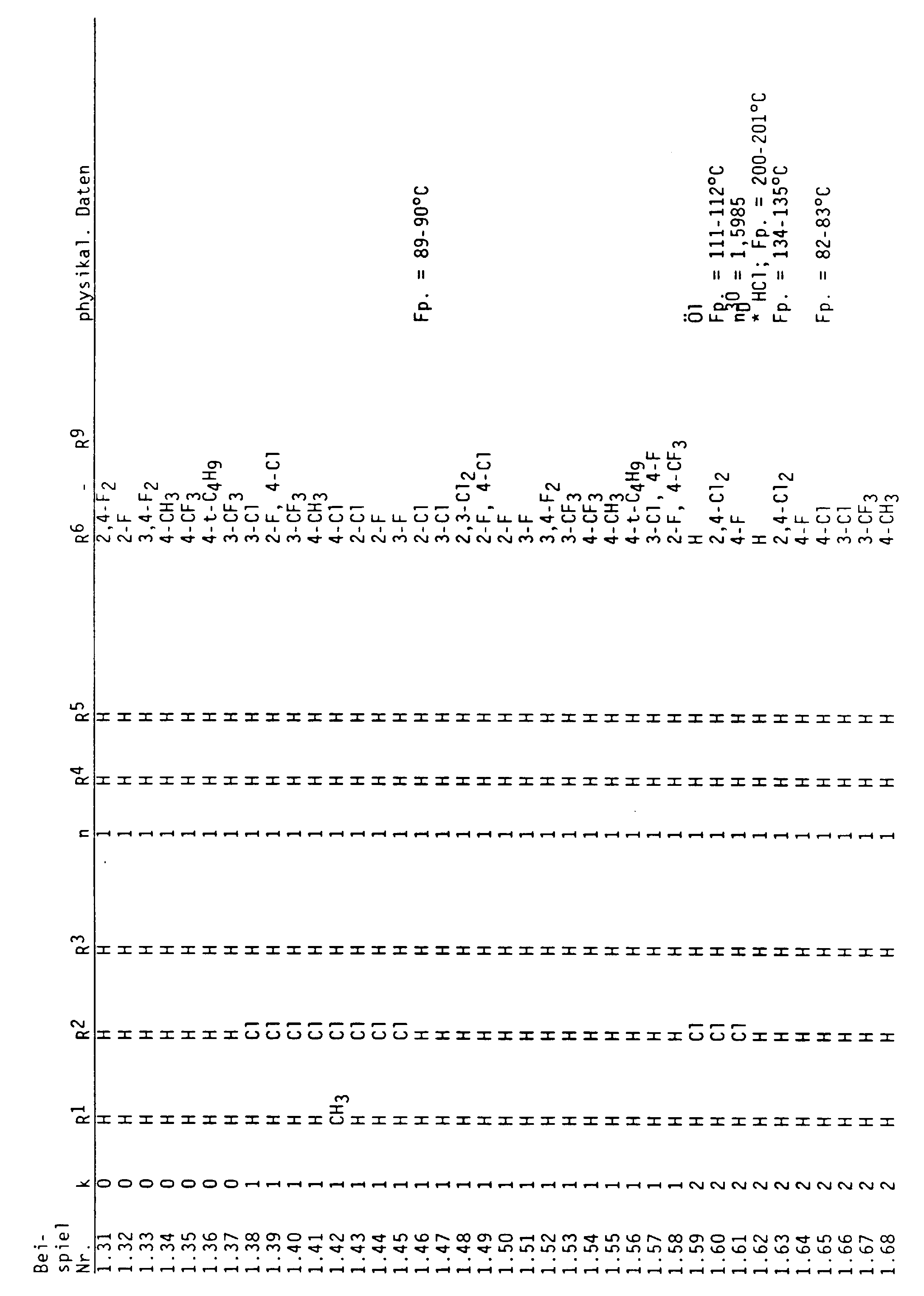

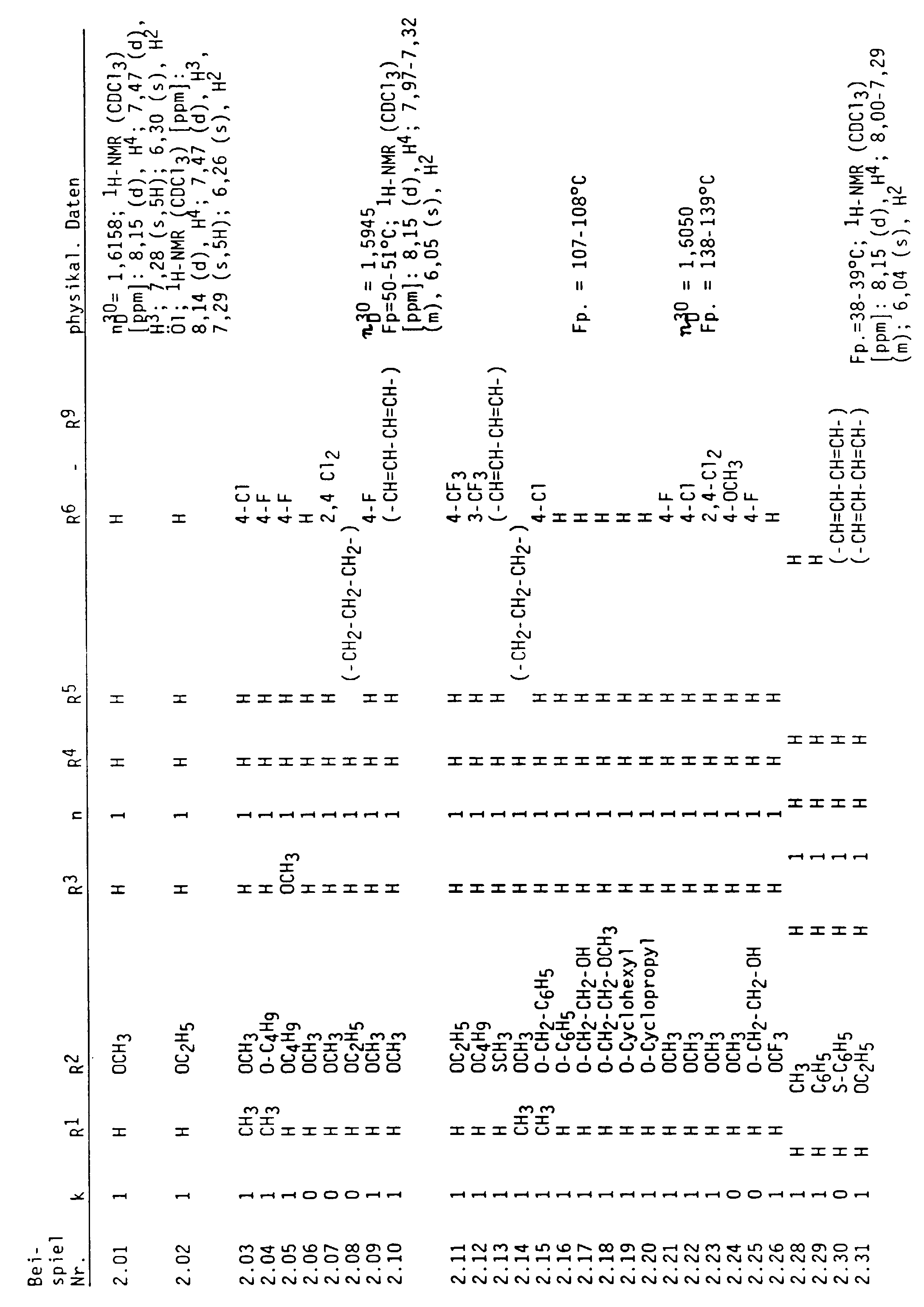

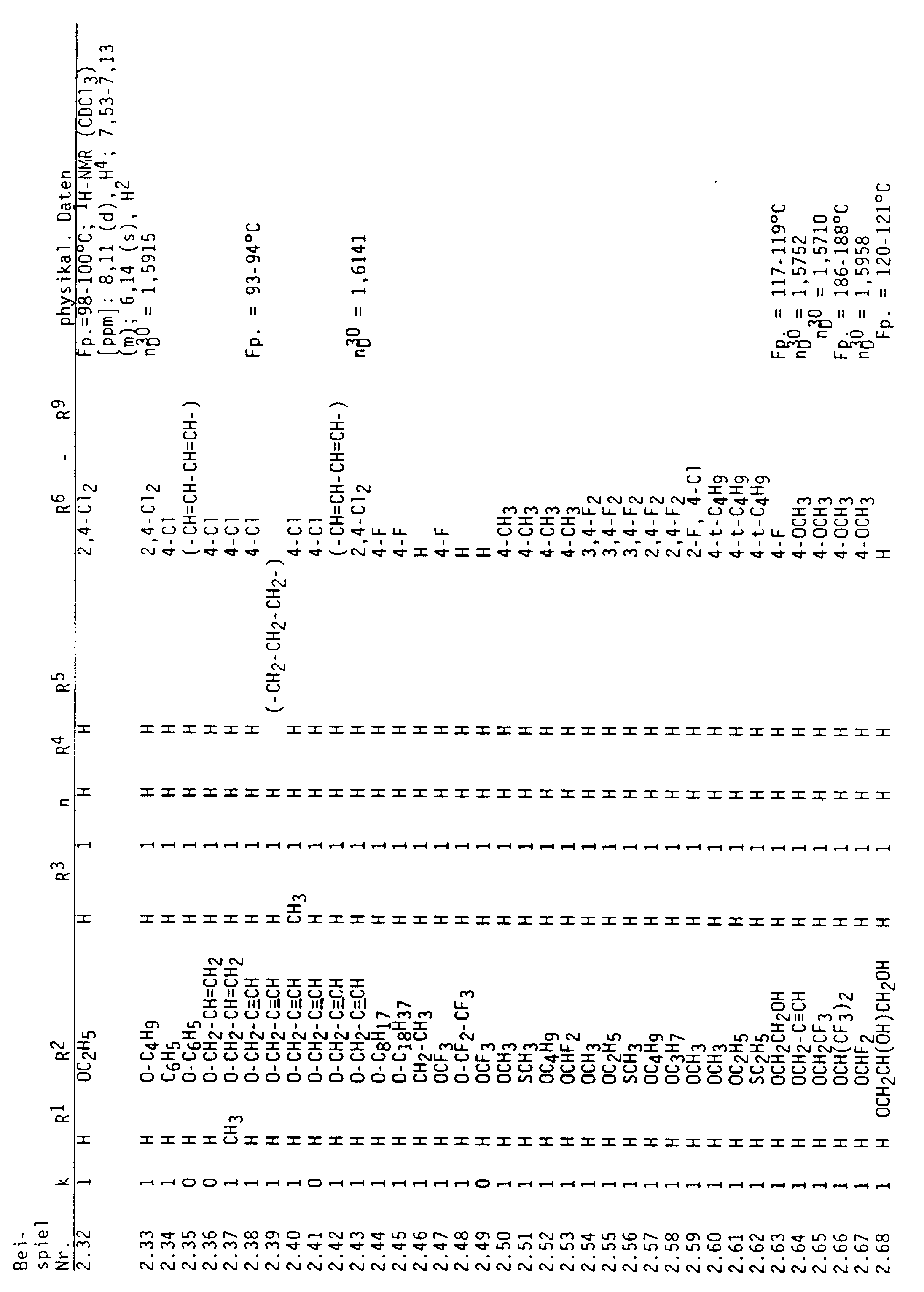

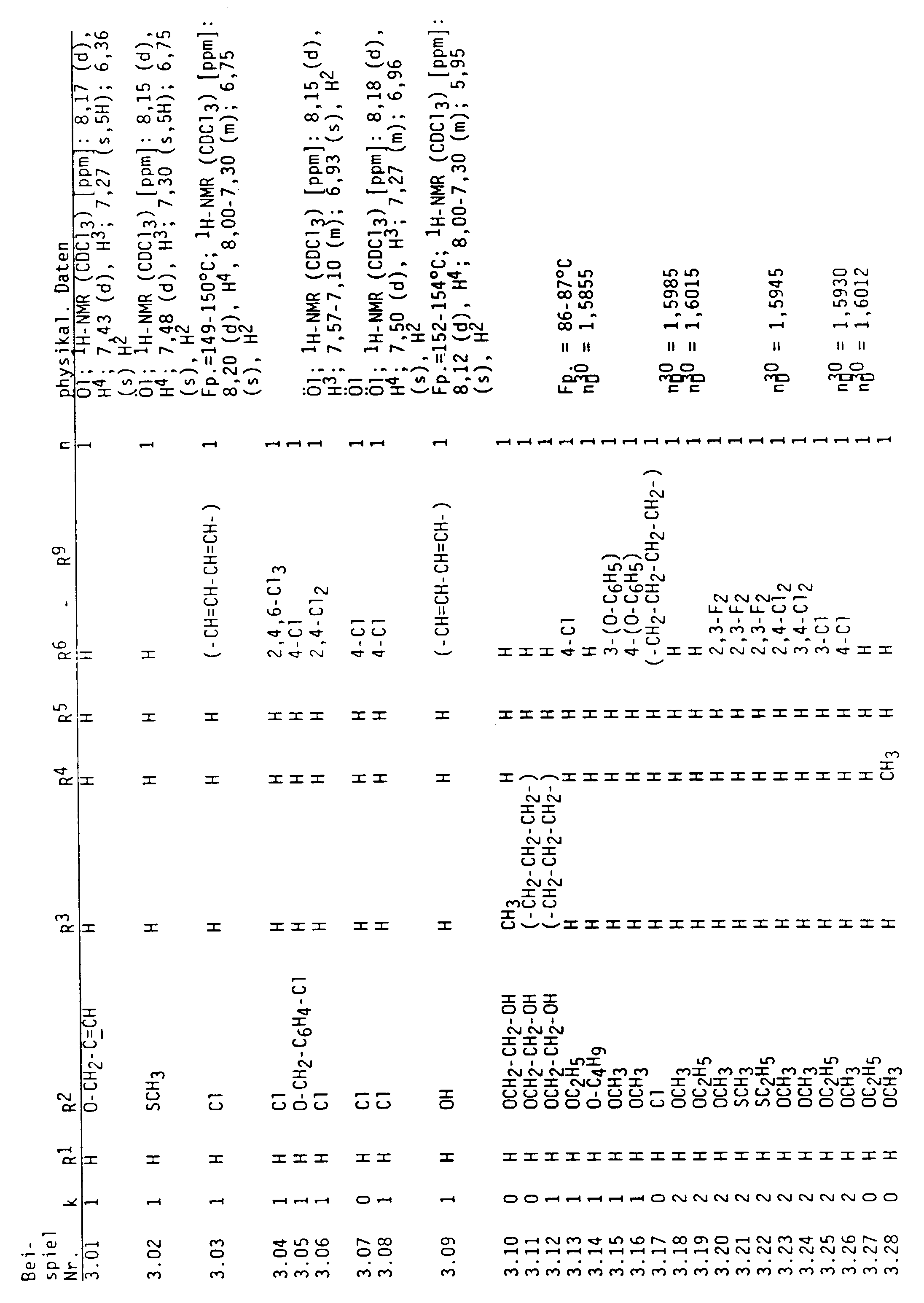

### C Biologische Beispiele

### Beispiel 1

Etwa 5 Wochen alte Reispflanzen der Sorte "Ballila" wurden mit den unten angegebenen Konzentrationen der beanspruchten Verbindungen behandelt. Nach Antrocknen des Spritzbelages wurden die Pflanzen mit einer Sporensuspension von Piricularia oryzae gleichmäßig inokuliert und 48 h in eine dunkel gehaltene Klimakammer mit einer Temperatur von 25°C und 100 % rel. Luftfeuchte gestellt. Danach wurden die Reispflanzen in einem Gewächshaus mit einer Temperatur von 25°C und 80 % rel. Luftfeuchte weiterkultiviert. Nach 5 Tagen erfolgte die Befallsauswertung. Der Befallsgrad wurde in % befallener Blattfläche im Vergleich zu unbehandelten, infizierten Kontrollpflanzen ausgedrückt. Die Ergebnisse sind in Tabelle 1 zusammengestellt.

**Tabelle 1**

| Verbindung gemäß Beispiel | mit Piricularia oryzae befallene Blattfläche in % bei mg Wirkstoff/Liter Spritzbrühe |
|---|---|
| 1.01 | 0 |
| 2.01 | 0 |
| 1.20 | 0 |
| 1.21 | 0 |
| 2.67 | 0 |
| 1.05 | 0 |
| 1.24 | 0 |
| 1.62 | 0 |
| unbehandelte, infizierte Pflanzen | 100 |

### Beispiel 2

Ca. 14 Tage alte Ackerbohnen der Sorten "Herz Freya" oder "Frank's Ackerperle" wurden mit wäßrigen Suspensionen der beanspruchten Verbindung tropfnaß behandelt.

Nach Antrocknen des Spritzbelages wurden die Pflanzen mit einer Sporensuspension (1,5 Mio. Sporen/ml) von Botrytis cinerea inokuliert. Die Pflanzen wurden in einer Klimakammer bei 20 - 22°C und ca. 99 % rel. Luftfeuchte weiterkultiviert. Die Infektion der Pflanzen äußert sich in der Bildung schwarzer Flecken auf den Blättern und Stengeln. Die Auswertung der Versuche erfolgte ca. 1 Woche nach Inokulation.

Der Befallsgrad wird ausgedrückt in % befallener Blattfläche, bezogen auf unbehandelte, infizierte Kontrollpflanzen (= 100 %). Das Ergebnis ist in Tabelle 2 wiedergegeben.

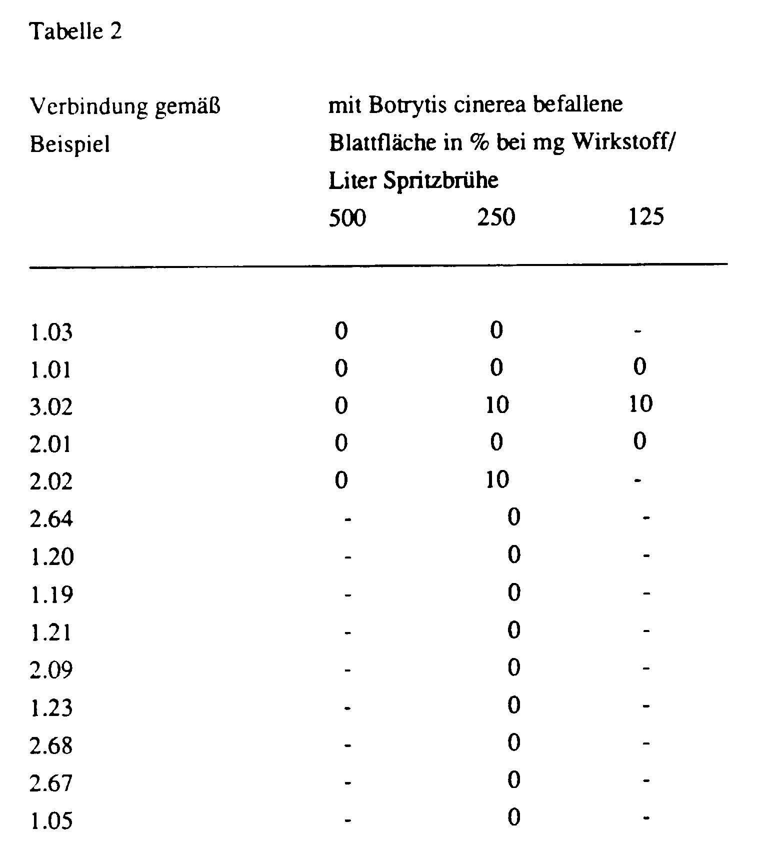

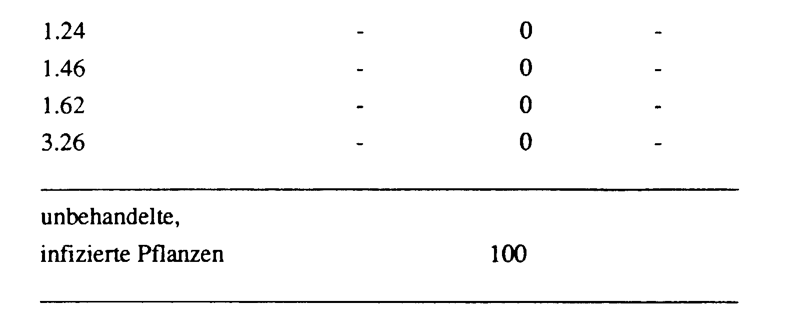

### Beispiel 3

Weizenpflanzen der Sorte "Jubilar" wurden im 2-Blatt-Stadium mit wäßrigen Suspensionen der in Tabelle 2 angegebenen Präparate tropfnaß behandelt.

Nach dem Antrocknen des Spritzbelages wurden die Pflanzen mit einer wäßrigen Pyknosporen-Suspension von Leptosphaeria nodorum inokuliert und mehrere Stunden bei 100 % rel. Luftfeuchte in einer Klimakammer inkubiert. Bis zur Symptomausprägung wurden die Pflanzen im Gewächshaus bei ca. 90 % rel. Luftfeuchte weiterkultiviert.

Der Befallsgrad wurde in % befallener Blattfläche im Vergleich zu unbehandelten infizierten Kontrollpflanzen ausgedrückt und wird in Tabelle 3 wiedergegeben.

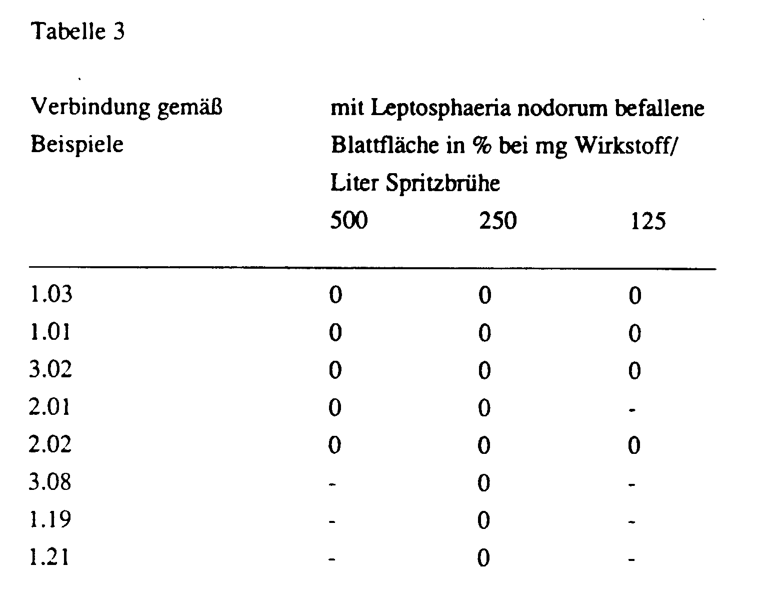

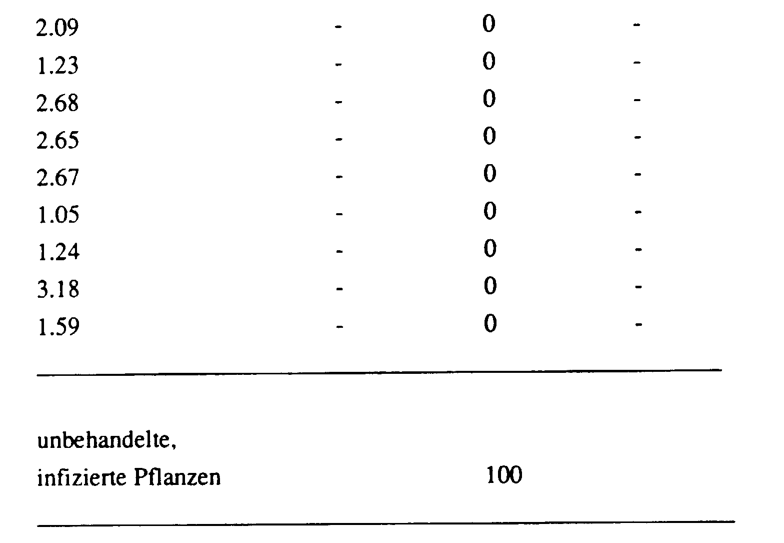

### Beispiel 4

Gerstenpflanzen der Sorte "Igri" wurden im 2-Blatt-Stadium mit einer wäßrigen Suspension der beanspruchten Verbindungen tropfnaß behandelt.

Nach dem Antrocknen des Spritzbelages wurden die Pflanzen mit einer wäßrigen Sporensuspension von Pyrenophora teres inokuliert und für 16 h in einer Klimakammer bei 100 % rel. Luftfeuchte inkubiert. Anschließend wurden die infizierten Pflanzen im Gewächshaus bei 25°C und 80 % rel. Luftfeuchte weiterkultiviert.

Ca. 1 Woche nach Inokulation wurde der Befall ausgewertet. Der Befallsgrad wurde in % befallener Blattfläche im Vergleich zur unbehandelten, infizierten Kontrolle bonitiert und ist in Tabelle 4 wiedergegeben.

**Tabelle 4**

| Verbindung gemäß Beispiel | mit Pyrenophora teres befallene Blattfläche in % bei mg Wirkstoff/Liter Spritzbrühe | | |
|---|---|---|---|
| | 500 | 250 | 125 |
| 1.03 | 0 | 0 | 0 |
| 1.01 | 0 | 0 | 0 |
| 2.02 | 0 | 0 | 0 |
| 2.64 | - | 0 | - |
| 2.22 | - | 0 | - |
| 1.19 | - | 0 | - |
| 1.21 | - | 0 | - |
| 2.09 | - | 0 | - |
| 1.20 | - | 0 | - |
| 2.63 | - | 0 | - |
| 2.17 | - | 0 | - |
| 1.23 | - | 0 | - |
| 2.68 | - | 0 | - |
| 2.67 | - | 0 | - |
| 1.05 | - | 0 | - |
| 1.24 | - | 0 | - |
| 1.62 | - | 0 | - |
| 3.18 | - | 0 | - |
| 3.26 | - | 0 | - |
| unbehandelte, infizierte Pflanzen | | 100 | |

## Patentansprüche (Patentansprüche für folgende(n) Vertragsstaat(en): AT, BE, CH, DE, FR, GB, IT, LI, NL, SE)

1. Pyrimidin-Derivate der Formel I 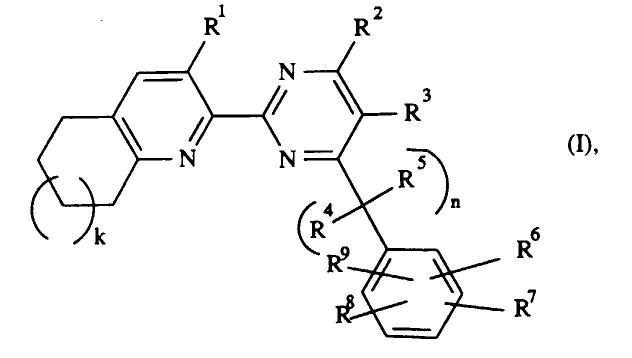 worin
R¹ = Wasserstoff, (C₁-C₆)Alkyl, (C₁-C₆)Alkoxy, Phenyl-(C₁-C₄)alkyl, wobei der Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
R² = Wasserstoff, Hydroxy, (C₂-C₄)Alkenyl-(C₁-C₄)alkyl, (C₂-C₄)Alkinyl-(C₁-C₄)alkyl, (C₁-C₆)Alkyl, (C₁-C₄)Alkoxy-(C₁-C₄)alkyl, (C₂-C₆)Alkenyl, (C₂-C₆)Alkinyl, (C₃-C₇)Cycloalkyl, (C₃-C₇)Cycloalkyl-(C₁-C₄)alkyl, wobei die beiden letztgenannten Reste im Cycloalkylteil bis zu dreifach durch (C₁-C₄)Alkyl substituiert sein können, Phenyl, Phenoxy-(C₁-C₄)alkyl, Phenyl-(C₁-C₄)alkyl, wobei die drei letztgenannten Reste im Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, (C₁-C₁₂)Alkoxy, (C₂-C₆)Alkenyl-(C₁-C₄)alkoxy, (C₂-C₆)Alkinyl-(C₁-C₄)alkoxy, (C₃-C₇)Cycloalkoxy, (C₁-C₆)Alkoxy-(C₁-C₆)alkoxy, Hydroxy-(C₁-C₆)alkoxy, (C₁-C₆)Haloalkoxy, (C₁-C₆)Alkylthio, (C₂-C₆)Alkenyl-(C₁-C₄)alkylthio, (C₂-C₆)Alkinyl-(C₁-C₄)alkylthio, (C₃-C₇)Cycloalkylthio, Phenoxy, Phenylthio, Phenyl-(C₁-C₆)alkoxy, Phenyl-(C₁-C₆)alkylthio, Phenyl-(C₁-C₂)alkoxy-(C₁-C₄)alkoxy, wobei der Phenylring in den fünf letztgenannten Resten bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy sowie einfach durch Nitro oder Cyano substituiert sein kann,
R³ = Wasserstoff, (C₁-C₆)Alkyl, (C₁-C₄)Haloalkyl, (C₁-C₄)Alkoxy, (C₁-C₄)Alkylthio, Halogen, Phenyl, Phenyl-(C₁-C₄)alkyl, wobei der Phenylrest der drei letztgenannten Reste bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy sowie einfach durch Nitro oder Cyano substituiert sein kann,
R⁴, R⁵ = unabhängig voneinander Wasserstoff, (C₁-C₆)Alkyl, oder
R³ mit R⁴ oder R⁵ Bestandteil eines maximal ungesättigten 1-gliedrigen Ringes mit 1 = 3 bis 8,
R⁶, R⁷, R⁸, R⁹ = unabhängig voneinander Wasserstoff, Halogen, Nitro, Cyano, (C₁-C₆)Alkyl, (C₁-C₆)Alkoxy, (C₁-C₆)Alkylthio, (C₁-C₆)Haloakyl, (C₁-C₆)Haloalkoxy, Aryl, Aryloxy, wobei die beiden letztgenannten Reste im Arylrest bis zu dreifach durch Halogen, (C₁-C₄)Alkyl, (C₁-C₄)Alkoxy, (C₁-C₄)Haloalkyl oder (C₁-C₄)Haloalkoxy substituiert sein können,
zwei der Reste R⁶ bis R⁹ gegebenenfalls Bestandteil eines ungesättigten oder gesättigten m-gliedrigen Ringes mit m = 5 oder 6,
k = 0, 1 oder 2,
n = 1 oder 2 und
Halo = ein- oder mehrfach durch Halogenatome substituiert bedeuten,
sowie deren Säureadditionssalze.

2. Verbindungen der Formel I gemäß Anspruch 1, dadurch gekennzeichnet, daß
R¹ = Wasserstoff, (C₁-C₄)Alkyl, (C₁-C₆)Alkoxy,
R² = Wasserstoff, Hydroxy, (C₁-C₄)Alkyl, (C₂-C₄)Alkenyl-(C₁-C₂)alkyl, (C₂-C₄)Alkinyl-(C₁-C₂)alkyl, (C₁-C₄)Alkoxy-(C₁-C₄)alkyl, (C₂-C₄)Alkenyl, (C₂-C₄)Alkinyl, (C₃-C₆)Cycloalkyl, (C₃-C₆)Cycloalkyl -(C₁-C₂)alkyl, wobei die beiden letztgenannten Reste im Cycloalkylteil bis zu dreifach durch (C₁-C₄)Alkyl substituiert sein können, Phenyl, Phenoxy-(C₁-C₂)alkyl,Phenyl-(C₁-C₂)alkyl, wobei die drei letztgenannten Reste im Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, (C₁-C₁₂)Alkoxy, (C₂-C₄)Alkenyl-(C₁-C₂)alkoxy, (C₂-C₄)Alkinyl-(C₁-C₂)alkoxy, (C₃-C₆)Cycloalkyloxy, (C₁-C₄)Alkoxy-(C₁-C₄)alkoxy, Hydroxy-(C₁-C₄)alkoxy, (C₁-C₆)Haloalkoxy, (C₁-C₆)Alkylthio, (C₂-C₆)Alkenyl-(C₁-C₂)alkylthio, (C₂-C₄)Alkinyl-(C₁-C₂)alkylthio, (C₃-C₆)Cycloalkylthio, Phenoxy, Phenylthio, Phenyl-(C₁-C₂)alkoxy, Phenyl-(C₁-C₂)alkylthio, Phenyl-(C₁-C₂)alkoxy-(C₁-C₄)alkoxy, wobei der Phenylring in den vier letztgenannten Resten bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
R³ = Wasserstoff, (C₁-C₄)Alkyl, (C₁-C₄)Haloalkyl, (C₁-C₄)Alkoxy, (C₁-C₄)Alkylthio, Halogen, Phenyl, Phenyl-(C₁ -C₂)alkyl,
R⁴, R⁵ = unabhängig voneinander Wasserstoff, (C₁-C₂)Alkyl oder
R³ mit R⁴ oder R⁵ Bestandteil eines maximal ungesättigten 1-gliedrigen Ringes mit 1 = 5 oder 6,
R⁶, R⁷, R⁸, R⁹ = unabhängig voneinander Wasserstoff, Halogen, Nitro, Cyano, (C₁-C₄)Alkyl, (C₁-C₄)Alkoxy, (C₁-C₄)Haloalkyl, (C₁-C₄)Haloalkoxy, Phenyl, Phenyloxy, wobei die beiden letztgenannten Reste im Phenylrest bis zu dreifach durch Halogen, (C₁-C₄)Alkyl, (C₁-C₄)Alkoxy, (C₁-C₄)Haloalkyl oder (C₁-C₄)Haloalkoxy substituiert sein können,
zwei der Reste R⁶ bis R⁹ gegebenenfalls Bestandteil eines ungesättigten oder gesättigten m-gliedrigen Ringes mit m = 5 oder 6,
k = 0, 1 oder 2,
n = 1 oder 2 und
Halo = ein- oder mehrfach durch Halogenatome substituiert bedeuten,
sowie deren Säureadditionssalze.

3. Verfahren zur Herstellung von Verbindungen der Formel I gemäß Anspruch 1 oder 2, dadurch gekennzeichnet, daß man
a) für Verbindungen mit R² = Wasserstoff ein entsprechendes Halopyrimidin der Formel I mit R² = Halogen und allen übrigen Substituenten in den genannten Bedeutungen reduktiv dehalogeniert oder
b) für Verbindungen der Formel 1, worin R² für einen mit dem Suffix "oxy" oder "thio" endenden, wie im Anspruch 1 bei R² definierten organischen Rest steht, ein entsprechendes Halopyrimidin der Formel I mit R² = Halogen mit einer Alkalimetallverbindung der Formel II
R² - Y (II)
worin R² die obengenannte Bedeutung besitzt und Y für ein Alkalimetall steht, umsetzt, oder
c) für Verbindungen der Formel I, worin R² einen nicht mit dem Suffix "oxy" oder "thio" endenden, wie im Anspruch 1 bei R² definierten organischen Rest bedeutet, ein entsprechendes Halopyrimidin der Formel I mit R² = Halogen mit einer Grignardverbindung der Formel III
R² - MgX (III),
worin R² die obengenannte Bedeutung besitzt und X = Halogen bedeutet, in Gegenwart von Nickel-Phosphin-Komplexen umsetzt.

4. Fungizide Mittel, dadurch gekennzeichnet, daß sie eine wirksame Menge einer Verbindung der Formel I gemäß Anspruch 1 oder 2 enthalten.

5. Verwendung von Verbindungen der Formel I gemäß Anspruch 1 oder 2 zur Bekämpfung von Schadpilzen.

6. Verfahren zur Bekämpfung von Schadpilzen, dadurch gekennzeichnet, daß man auf diese oder die von ihnen befallenen Pflanzen, Flächen oder Substrate eine wirksame Menge einer Verbindung der Formel I gemäß Anspruch 1 oder 2 appliziert.

## Patentansprüche (Patentansprüche für folgende(n) Vertragsstaat(en): ES)

1. Verfahren zur Herstellung von Verbindungen der Formel I 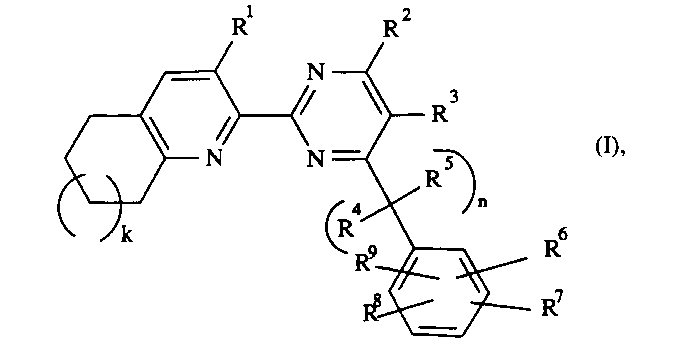 worin
R¹ = Wasserstoff, (C₁-C₆)Alkyl, (C₁-C₆)Alkoxy, Phenyl-(C₁-C₄)alkyl, wobei der Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
R² = Wasserstoff, Hydroxy, (C₂-C₄)Alkenyl-(C₁-C₄)alkyl, (C₂-C₄)Alkinyl-(C₁-C₄)alkyl, (C₁-C₆)Alkyl, (C₁-C₄)Alkoxy-(C₁-C₄)alkyl, (C₂-C₆)Alkenyl, (C₂-C₆)Alkinyl, (C₃-C₇)Cycloalkyl, (C₃-C₇)Cycloalkyl-(C₁-C₄)alkyl, wobei die beiden letztgenannten Reste im Cycloalkylteil bis zu dreifach durch (C₁-C₄)Alkyl substituiert sein können, Phenyl, Phenoxy-(C₁-C₄)alkyl, Phenyl-(C₁-C₄)alkyl, wobei die drei letztgenannten Reste im Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, (C₁-C₁₂)Alkoxy, (C₂-C₆)Alkenyl-(C₁-C₄)alkoxy, (C₂-C₆)Alkinyl-(C₁-C₄)alkoxy, (C₃-C₇)Cycloalkoxy, (C₁-C₆)Alkoxy-(C₁-C₆)alkoxy, Hydroxy-(C₁-C₆)alkoxy, (C₁-C₆)Haloalkoxy, (C₁-C₆)Alkylthio, (C₂-C₆)Alkenyl-(C₁-C₄)alkylthio, (C₂-C₆)Alkinyl-(C₁-C₄)alkylthio, (C₃-C₇)Cycloalkylthio, Phenoxy, Phenylthio, Phenyl-(C₁-C₆)alkoxy, Phenyl-(C₁-C₆)alkylthio, Phenyl-(C₁-C₂)alkoxy-(C₁-C₄)alkoxy, wobei der Phenylring in den fünf letztgenannten Resten bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy sowie einfach durch Nitro oder Cyano substituiert sein kann,
R³ = Wasserstoff, (C₁-C₆)Alkyl, (C₁-C₄)Haloalkyl, (C₁-C₄)Alkoxy, (C₁-C₄)Alkylthio, Halogen, Phenyl, Phenyl-(C₁-C₄)alkyl, wobei der Phenylrest der drei letztgenannten Reste bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy sowie einfach durch Nitro oder Cyano substituiert sein kann,
R⁴, R⁵ = unabhängig voneinander Wasserstoff, (C₁-C₆)Alkyl, oder
R³ mit R⁴ oder R⁵ Bestandteil eines maximal ungesättigten 1-gliedrigen Ringes mit 1 = 3 bis 8,
R⁶, R⁷, R⁸, R⁹ = unabhängig voneinander Wasserstoff, Halogen, Nitro, Cyano, (C₁-C₆)Alkyl, (C₁-C₆)Alkoxy, (C₁-C₆)Alkylthio, (C₁-C₆)Haloalkyl, (C₁-C₆)Haloalkoxy, Aryl, Aryloxy, wobei die beiden letztgenannten Reste im Arylrest bis zu dreifach durch Halogen, (C₁-C₄)Alkyl, (C₁-C₄)Alkoxy, (C₁-C₄)Haloalkyl oder (C₁-C₄)Haloalkoxy substituiert sein können,
zwei der Reste R⁶ bis R⁹ gegebenenfalls Bestandteil eines ungesättigten oder gesättigten m-gliedrigen Ringes mit m = 5 oder 6,
k = 0, 1 oder 2,
n = 1 oder 2 und
Halo = ein- oder mehrfach durch Halogenatome substituiert bedeuten,
sowie deren Säureadditionssalze, dadurch gekennzeichnet, daß man
a) für Verbindungen mit R² = Wasserstoff ein entsprechendes Halpyrimidin der Formel I mit R² = Halogen und allen übrigen Substituenten in den genannten Bedeutungen reduktiv dehalogeniert oder
b) für Verbindungen der Formel 1, worin R² für einen mit dem Suffix "oxy" oder "thio" endenden, wie eingangs bei R² definierten organischen Rest steht, ein entsprechendes Halpyrimidin der Formel I mit R² = Halogen mit einer Alkalimetallverbindung der Formel II
R² - Y (II),
worin R² die obengenannte Bedeutung besitzt und Y für ein Alkalimetall steht, umsetzt, oder
c) für Verbindungen der Formel I, worin R² einen nicht mit dem Suffix "oxy" oder "thio" endenden, wie eingangs bei R² definierten organischen Rest bedeutet, ein entsprechendes Halopyrimidin der Formel I mit R² = Halogen mit einer Grignardverbindung der Formel III
R² - MgX (III)
worin R² die obengenannte Bedeutung besitzt und X = Halogen bedeutet, in Gegenwart von Nickel-Phosphin-Komplexen umsetzt.

2. Verfahren gemäß Anspruch 1, dadurch gekennzeichnet, daß in Formel I
R¹ = Wasserstoff, (C₁-C₄)Alkyl, (C₁-C₆)Alkoxy,
R² = Wasserstoff, Hydroxy, (C₁-C₄)Alkyl, (C₂-C₄)Alkenyl-(C₁-C₂)alkyl, (C₂-C₄)Alkinyl-(C₁-C₂)alkyl, (C₁-C₄)Alkoxy-(C₁-C₄)alkyl, (C₂-C₄)Alkenyl, (C₂-C₄)Alkinyl, (C₃-C₆)Cycloalkyl, (C₃-C₆)Cycloalkyl-(C₁-C₂)alkyl, wobei die beiden letztgenannten Reste im Cycloalkylteil bis zu dreifach durch (C₁-C₄)Alkyl substituiert sein können, Phenyl, Phenoxy-(C₁-C₂)alkyl, Phenyl-(C₁-C₂)alkyl, wobei die drei letztgenannten Reste im Phenylteil bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein können, (C₁-C₁₂)Alkoxy, (C₂-C₄)Alkenyl-(C₁-C₂)alkoxy, (C₂-C₄)Alkinyl-(C₁-C₂)alkoxy, (C₃-C₆)Cycloalkyloxy, (C₁-C₄)Alkoxy-(C₁-C₄)alkoxy, Hydroxy-(C₁-C₄)alkoxy, (C₁-C₆)Haloalkoxy, (C₁-C₆)Alkylthio, (C₂-C₆)Alkenyl-(C₁-C₂)alkylthio, (C₂-C₄)Alkinyl-(C₁-C₂)alkylthio, (C₃-C₆)Cycloalkylthio, Phenoxy, Phenylthio, Phenyl-(C₁-C₂)alkoxy, Phenyl-(C₁-C₂)alkylthio, Phenyl-(C₁-C₂)alkoxy-(C₁-C₄)alkoxy, wobei der Phenylring in den vier letztgenannten Resten bis zu dreifach durch Halogen, (C₁-C₄)Alkyl, (C₁-C₄)Alkoxy, (C₁-C₄)Alkylthio, (C₁-C₄)Haloalkyl oder (C₁-C₄)Haloalkoxy substituiert sein kann,
R³ = Wasserstoff, (C₁-C₄)Alkyl, (C₁-C₄)Haloalkyl, (C₁-C₄)Alkoxy, (C₁-C₄)Alkylthio, Halogen, Phenyl, Phenyl-(C₁ -C₂)alkyl,
R⁴, R⁵ = unabhängig voneinander Wasserstoff, (C₁-C₂)Alkyl oder
R³ mit R⁴ oder R⁵ Bestandteil eines maximal ungesättigten 1-gliedrigen Ringes mit 1 = 5 oder 6,
R⁶, R⁷, R⁸, R⁹ = unabhängig voneinander Wasserstoff, Halogen, Nitro, Cyano, (C₁-C₄)Alkyl, (C₁-C₄)Alkoxy, (C₁-C₄)Haloalkyl, (C₁-C₄)Haloalkoxy, Phenyl, Phenyloxy, wobei die beiden letztgenannten Reste im Phenylrest bis zu dreifach durch Halogen, (C₁-C₄)Alkyl, (C₁-C₄)Alkoxy, (C₁-C₄)Haloalkyl oder (C₁-C₄)Haloalkoxy substituiert sein können,
zwei der Reste R⁶ bis R⁹ gegebenenfalls Bestandteil eines ungesättigten oder gesättigten m-gliedrigen Ringes mit m = 5 oder 6,
k = 0, 1 oder 2,
n = 1 oder 2 und
Halo = ein- oder mehrfach durch Halogenatome substituiert bedeuten.

3. Fungizide Mittel, dadurch gekennzeichnet, daß sie eine wirksame Menge einer Verbindung der Formel I gemäß Anspruch 1 oder 2 enthalten.

4. Verwendung von Verbindungen der Formel I gemäß Anspruch 1 oder 2 zur Bekämpfung von Schadpilzen.

5. Verfahren zur Bekämpfung von Schadpilzen, dadurch gekennzeichnet, daß man auf diese oder die von ihnen befallenen Pflanzen, Flächen oder Substrate eine wirksame Menge einer Verbindung der Formel I gemäß Anspruch 1 oder 2 appliziert.

## Claims (Claims for the following Contracting State(s): AT, BE, CH, DE, FR, GB, IT, LI, NL, SE)

1. A pyrimidine derivative of the formula I 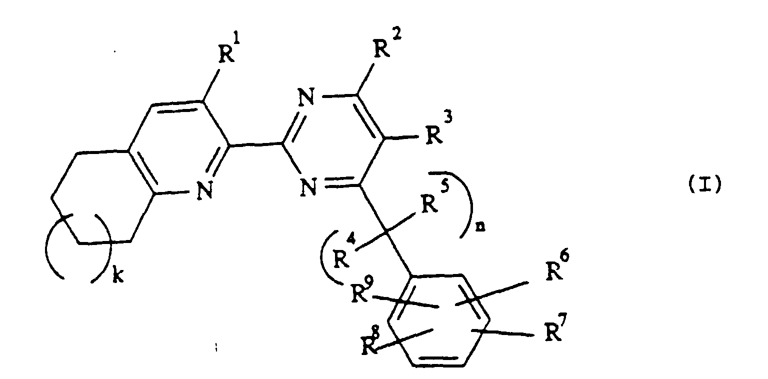 in which
R¹ is hydrogen, (C₁-C₆)alkyl, (C₁-C₆)alkoxy, phenyl-(C₁-C₄)alkyl, it being possible for the phenyl moiety to be up to trisubstituted by halogen, (C₁-C₄)alkyl, (C₁-C₄)alkoxy, (C₁-C₄)alkylthio, (C₁-C₄)haloalkyl or (C₁-C₄)haloalkoxy,
R² is hydrogen, hydroxyl, (C₂-C₄)alkenyl-(C₁-C₄)alkyl, (C₂-C₄)alkynyl-(C₁-C₄)alkyl, (C₁-C₆)alkyl, (C₁-C₄)-alkoxy-(C₁-C₄)alkyl, (C₂-C₆)alkenyl, (C₂-C₆)alkynyl, (C₃-C₇)cycloalkyl, (C₃-C₇)cycloalkyl-(C₁-C₄)alkyl, it being possible for the two last-mentioned radicals to be up to trisubstituted in the cycloalkyl moiety by (C₁-C₄)alkyl, or is phenyl, phenoxy-(C₁-C₄)alkyl, phenyl-(C₁-C₄)alkyl, it being possible for the three last-mentioned radicals to be up to trisubstituted in the phenyl moiety by halogen, (C₁-C₄)alkyl, (C₁-C₄)alkoxy, (C₁-C₄)alkylthio, (C₁-C₄)haloalkyl or (C₁-C₄)haloalkoxy, or is (C₁-C₁₂)alkoxy, (C₂-C₆)alkenyl- (C₁-C₄)alkoxy, (C₂-C₆)alkynyl- (C₁-C₄)alkoxy, (C₃-C₇)cycloalkoxy,(C₁-C₆)alkoxy-(C₁-C₆)alkoxy, hydroxy-(C₁-C₆)alkoxy, (C₁-C₆)haloalkoxy, (C₁-C₆)-alkylthio, (C₂-C₆)alkenyl-(C₁-C₄)alkylthio, (C₂-C₆)-alkynyl-(C₁-C₄)alkylthio, (C₃-C₇)cycloalkylthio, phenoxy, phenylthio, phenyl-(C₁-C₆)alkoxy, phenyl-(C₁-C₆)alkylthio,phenyl-(C₁-C₂)alkoxy-(C₁-C₄)alkoxy, it being possible for the phenyl ring in the five last-mentioned radicals to be up to trisubstituted by halogen, (C₁-C₄)alkyl, (C₁-C₄)alkoxy, (C₁-C₄)-alkylthio, (C₁-C₄)haloalkyl or (C₁-C₄)haloalkoxy or monosubstituted by nitro or cyano,
R³ is hydrogen, (C₁-C₆)alkyl, (C₁-C₄)haloalkyl, (C₁-C₄)-alkoxy, (C₁-C₄)alkylthio, halogen, phenyl, phenyl-(C₁-C₄)alkyl, it being possible for the phenyl radical of the three last-mentioned radicals to be up to trisubstituted by halogen, (C₁-C₄)alkyl, (C₁-C₄)alkoxy, (C₁-C₄)alkylthio, (C₁-C₄)haloalkyl or (C₁-C₄)haloalkoxy or monosubstituted by nitro or cyano,
R⁴ and R⁵ independently of one another are hydrogen or (C₁-C₆)alkyl, or
R³ together with R⁴ or R⁵ is a component of a maximally unsaturated 1-membered ring where 1 is 3 to 8,
R⁶, R⁷, R⁸ and R⁹ independently of one another are hydrogen, halogen, nitro, cyano, (C₁-C₆)alkyl, (C₁-C₆)alkoxy, (C₁-C₆)alkylthio, (C₁-C₆)haloalkyl, (C₁-C₆)haloalkoxy, aryl, aryloxy, it being possible for the two last-mentioned radicals to be up to trisubstituted in the aryl radical by halogen, (C₁-C₄)alkyl, (C₁-C₄)alkoxy, (C₁-C₄)haloalkyl or (C₁-C₄)haloalkoxy,
two of the radicals R⁶ to R⁹ may form a component of an unsaturated or saturated m-membered ring where m is 5 or 6,
k is 0, 1 or 2,
n is 1 or 2 and
halo has the meaning of monosubstituted or polysubstituted by halogen atoms,
and the acid addition salts thereof.

2. A compound of the formula I as claimed in claim 1, wherein
R¹ is hydrogen, (C₁-C₄)alkyl, (C₁-C₆)alkoxy,
R² is hydrogen, hydroxyl, (C₁-C₄)alkyl, (C₂-C₄)alkenyl-(C₁-C₂)alkyl, (C₂-C₄)alkynyl-(C₁-C₂)alkyl, (C₁-C₄)-alkoxy-(C₁-C₄)alkyl, (C₂-C₄)alkenyl, (C₂-C₄)alkynyl, (C₃-C₆)cycloalkyl, (C₃-C₆)cycloalkyl-(C₁-C₂)alkyl, it being possible for the two last-mentioned radicals to be up to trisubstituted in the cycloalkyl moiety by (C₁-C₄)alkyl, or is phenyl, phenoxy-(C₁-C₂)alkyl, phenyl-(C₁-C₂)alkyl, it being possible for the three last-mentioned radicals to be up to trisubstituted in the phenyl moiety by halogen, (C₁-C₄)alkyl, (C₁-C₄)alkoxy, (C₁-C₄)alkylthio, (C₁-C₄)haloalkyl or (C₁-C₄)haloalkoxy, or is (C₁-C₁₂)alkoxy, (C₂-C₄)alkenyl-(C₁-C₂)alkoxy, (C₂-C₄)alkynyl-(C₁-C₂)alkoxy, (C₃-C₆)cycloalkyloxy, (C₁-C₄)alkoxy- (C₁-C₄)alkoxy, hydroxy-(C₁-C₄)alkoxy, (C₁-C₆)haloalkoxy, (C₁-C₆)-alkylthio, (C₂-C₆)alkenyl-(C₁-C₂)alkylthio, (C₂-C₄)-alkynyl-(C₁-C₂)alkylthio, (C₃-C₆)cycloalkylthio, phenoxy, phenylthio, phenyl-(C₁-C₂)alkoxy, phenyl-(C₁-C₂)alkylthio,phenyl-(C₁-C₂)alkoxy-(C₁-C₄)alkoxy, it being possible for the phenyl ring in the four last-mentioned radicals to be up to trisubstituted by halogen, (C₁-C₄)alkyl, (C₁-C₄)alkoxy, (C₁-C₄)-alkylthio, (C₁-C₄)haloalkyl or (C₁-C₄)haloalkoxy,
R³ is hydrogen, (C₁-C₄)alkyl, (C₁-C₄)haloalkyl, (C₁-C₄)-alkoxy, (C₁-C₄)alkylthio, halogen, phenyl, phenyl-(C₁-C₂)alkyl,
R⁴ and R⁵ independently of one another are hydrogen or (C₁-C₂)alkyl or
R³ together with R⁴ or R⁵ is a component of a maximally unsaturated 1-membered ring where 1 is 5 or 6,
R⁶, R⁷, R⁸ and R⁹ independently of one another are hydrogen, halogen, nitro, cyano, (C₁-C₄)alkyl, (C₁-C₄)alkoxy, (C₁-C₄)haloalkyl, (C₁-C₄)haloalkoxy, phenyl, phenyloxy, it being possible for the two last-mentioned radicals to be up to trisubstituted in the phenyl radical by halogen, (C₁-C₄)alkyl, (C₁-C₄)alkoxy, (C₁-C₄)haloalkyl or (C₁-C₄)haloalkoxy,
two of the radicals R⁶ to R⁹ may form a component of an unsaturated or saturated m-membered ring where m is 5 or 6,
k is 0, 1 or 2,
n is 1 or 2 and
halo has the meaning of monosubstituted or polysubstituted by halogen atoms,
and the acid addition salts thereof.

3. A process for the preparation of a compound of the formula I as claimed in claim 1 or 2, which comprises
a) in the case of compounds where R² is hydrogen, subjecting a corresponding halopyrimidine of the formula I where R² is halogen and all other substituents have the abovementioned meanings to reductive dehalogenation, or
b) in the case of compounds of the formula I where R² is an organic radical ending with the suffix "oxy" or "thio", as defined for R² in claim 1, reacting a corresponding halopyrimidine of the formula I where R² is halogen with an alkali metal compound of the formula II
R²-Y (II)
in which R² has the abovementioned meaning and Y is an alkali metal, or
c) in the case of compounds of the formula I where R² is an organic radical not ending with the suffix "oxy" or "thio", as defined for R² in claim 1, reacting a corresponding halopyrimidine of the formula I where R² is halogen with a Grignard compound of the formula III
R²-MgX (III)
in which R² has the abovementioned meaning and X is halogen, in the presence of nickel phosphine complexes.

4. A fungicidal composition, which contains an effective amount of a compound of the formula I as claimed in claim 1 or 2.

5. The use of a compound of the formula I as claimed in claim 1 or 2 for controlling harmful fungi.

6. A method of controlling harmful fungi, which comprises applying an effective amount of a compound of the formula I as claimed in claim 1 or 2 to these harmful fungi or the plants, surfaces or substrates infected with them.

## Claims (Claims for the following Contracting State(s): ES)

1. A process for the preparation of a compound of the formula I 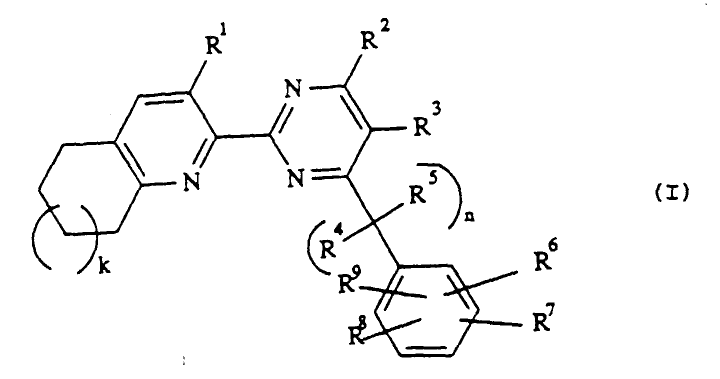 in which
R¹ is hydrogen, (C₁-C₆)alkyl, (C₁-C₆)alkoxy, phenyl-(C₁-C₄)alkyl, it being possible for the phenyl moiety to be up to trisubstituted by halogen, (C₁-C₄)alkyl, (C₁-C₄)alkoxy, (C₁-C₄)alkylthio, (C₁-C₄)haloalkyl or (C₁-C₄)haloalkoxy,
R² is hydrogen, hydroxyl, (C₂-C₄)alkenyl-(C₁-C₄)alkyl, (C₂-C₄)alkynyl-(C₁-C₄)alkyl, (C₁-C₆)alkyl, (C₁-C₄)-alkoxy-(C₁-C₄)alkyl, (C₂-C₆)alkenyl, (C₂-C₆)alkynyl, (C₃-C₇)cycloalkyl, (C₃-C₇)cycloalkyl-(C₁-C₄)alkyl, it being possible for the two last-mentioned radicals to be up to trisubstituted in the cycloalkyl moiety by (C₁-C₄)alkyl, or is phenyl, phenoxy-(C₁-C₄)alkyl, phenyl-(C₁-C₄)alkyl, it being possible for the three last-mentioned radicals to be up to trisubstituted in the phenyl moiety by halogen, (C₁-C₄)alkyl, (C₁-C₄)alkoxy, (C₁-C₄)alkylthio, (C₁-C₄)haloalkyl or (C₁-C₄)haloalkoxy, or is (C₁-C₁₂)alkoxy, (C₂-C₆)alkenyl-(C₁-C₄)alkoxy, (C₂-C₆)alkynyl-(C₁-C₄)alkoxy, (C₃-C₇)cycloalkoxy, (C₁-C₆)alkoxy-(C₁-C₆)alkoxy, hydroxy-(C₁-C₆)alkoxy, (C₁-C₆)haloalkoxy, (C₁-C₆)-alkylthio, (C₂-C₆)alkenyl-(C₁-C₄)alkylthio, (C₂-C₆)-alkynyl-(C₁-C₄)alkylthio, (C₃-C₇)cycloalkylthio, phenoxy, phenylthio, phenyl-(C₁-C₆)alkoxy, phenyl-(C₁-C₆)alkylthio,phenyl-(C₁-C₂)alkoxy-(C₁-C₄)alkoxy, it being possible for the phenyl ring in the five last-mentioned radicals to be up to trisubstituted by halogen, (C₁-C₄)alkyl, (C₁-C₄)alkoxy, (C₁-C₄)-alkylthio, (C₁-C₄)haloalkyl or (C₁-C₄)haloalkoxy or monosubstituted by nitro or cyano,
R³ is hydrogen, (C₁-C₆)alkyl, (C₁-C₄)haloalkyl, (C₁-C₄)-alkoxy, (C₁-C₄)alkylthio, halogen, phenyl, phenyl-(C₁-C₄)alkyl, it being possible for the phenyl radical of the three last-mentioned radicals to be up to trisubstituted by halogen, (C₁-C₄)alkyl, (C₁-C₄)alkoxy, (C₁-C₄)alkylthio, (C₁-C₄)haloalkyl or (C₁-C₄)haloalkoxy or monosubstituted by nitro or cyano,
R⁴ and R⁵ independently of one another are hydrogen or (C₁-C₆)alkyl, or
R³ together with R⁴ or R⁵ is a component of a maximally unsaturated 1-membered ring where I is 3 to 8,
R⁶, R⁷, R⁸ and R⁹ independently of one another are hydrogen, halogen, nitro, cyano, (C₁-C₆)alkyl, (C₁-C₆)alkoxy, (C₁-C₆)alkylthio, (C₁-C₆)haloalkyl, (C₁-C₆)haloalkoxy, aryl, aryloxy, it being possible for the two last-mentioned radicals to be up to trisubstituted in the aryl radical by halogen, (C₁-C₄)alkyl, (C₁-C₄)alkoxy, (C₁-C₄)haloalkyl or (C₁-C₄)haloalkoxy,
two of the radicals R⁶ to R⁹ may form a component of an unsaturated or saturated m-membered ring where m is 5 or 6,
k is 0, 1 or 2,
n is 1 or 2 and
halo has the meaning of monosubstituted or polysubstituted by halogen atoms,
and the acid addition salts thereof, which comprises
a) in the case of compounds where R² is hydrogen, subjecting a corresponding halopyrimidine of the formula I where R² is halogen and all other substituents have the abovementioned meanings to reductive dehalogenation, or
b) in the case of compounds of the formula I where R² is an organic radical ending with the suffix "oxy" or "thio", as defined for R² at the start, reacting a corresponding halopyrimidine of the formula I where R² is halogen with an alkali metal compound of the formula II
R²-Y (II)
in which R² has the abovementioned meaning and Y is an alkali metal, or
c) in the case of compounds of the formula I where R² is an organic radical not ending with the suffix "oxy" or "thio", as defined for R² at the start, reacting a corresponding halopyrimidine of the formula I where R² is halogen with a Grignard compound of the formula III
R²-MgX (III)
in which R² has the abovementioned meaning and X is halogen, in the presence of nickel phosphine complexes.

2. The process as claimed in claim 1, wherein in formula I
R¹ is hydrogen, (C₁-C₄)alkyl, (C₁-C₆)alkoxy,
R² is hydrogen, hydroxyl, (C₁-C₄)alkyl, (C₂-C₄)alkenyl-(C₁-C₂)alkyl, (C₂-C₄)alkynyl-(C₁-C₂)alkyl, (C₁-C₄)-alkoxy-(C₁-C₄)alkyl, (C₂-C₄)alkenyl, (C₂-C₄)alkynyl, (C₃-C₆)cycloalkyl, (C₃-C₆)cycloalkyl-(C₁-C₂)alkyl, it being possible for the two last-mentioned radicals to be up to trisubstituted in the cycloalkyl moiety by (C₁-C₄)alkyl, or is phenyl, phenoxy-(C₁-C₂)alkyl, phenyl-(C₁-C₂)alkyl, it being possible for the three last-mentioned radicals to be up to trisubstituted in the phenyl moiety by halogen, (C₁-C₄)alkyl, (C₁-C₄)alkoxy, (C₁-C₄)alkylthio, (C₁-C₄)haloalkyl or (C₁-C₄)haloalkoxy, or is (C₁-C₁₂)alkoxy, (C₂-C₄)alkenyl-(C₁-C₂)alkoxy, (C₂-C₄)alkynyl-(C₁-C₂)alkoxy, (C₃-C₆)cycloalkyloxy, (C₁-C₄)alkoxy-(C₁-C₄)alkoxy, hydroxy-(C₁-C₄)alkoxyl (C₁-C₆)haloalkoxy, (C₁-C₆)-alkylthio, (C₂-C₆)alkenyl-(C₁-C₂)alkylthio, (C₂-C₄)-alkynyl-(C₁-C₂)alkylthio, (C₃-C₆)cycloalkylthio, phenoxy, phenylthio, phenyl-(C₁-C₂)alkoxy, phenyl-(C₁-C₂)alkylthio,phenyl-(C₁-C₂)alkoxy-(C₁-C₄)alkoxy, it being possible for the phenyl ring in the four last-mentioned radicals to be up to trisubstituted by halogen, (C₁-C₄)alkyl, (C₁-C₄)alkoxy, (C₁-C₄)-alkylthio, (C₁-C₄)haloalkyl or (C₁-C₄)haloalkoxy,
R³ is hydrogen, (C₁-C₄)alkyl, (C₁-C₄)haloalkyl, (C₁-C₄)-alkoxy, (C₁-C₄)alkylthio, halogen, phenyl, phenyl-(C₁-C₂)alkyl,
R⁴ and R⁵ independently of one another are hydrogen or (C₁-C₂)alkyl or
R³ together with R⁴ or R⁵ is a component of a maximally unsaturated 1-membered ring where 1 is 5 or 6,
R⁶, R⁷, R⁸ and R⁹ independently of one another are hydrogen, halogen, nitro, cyano, (C₁-C₄)alkyl, (C₁-C₄)alkoxy, (C₁-C₄)haloalkyl, (C₁-C₄)haloalkoxy, phenyl, phenyloxy, it being possible for the two last-mentioned radicals to be up to trisubstituted in the phenyl radical by halogen, (C₁-C₄)alkyl, (C₁-C₄)alkoxy, (C₁-C₄)haloalkyl or (C₁-C₄)haloalkoxy,
two of the radicals R⁶ to R⁹ may form a component of an unsaturated or saturated m-membered ring where m is 5 or 6,
k is 0, 1 or 2,
n is 1 or 2 and
halo has the meaning of monosubstituted or polysubstituted by halogen atoms.

3. A fungicidal composition, which contains an effective amount of a compound of the formula I as claimed in claim 1 or 2.

4. The use of a compound of the formula I as claimed in claim 1 or 2 for controlling harmful fungi.

5. A method of controlling harmful fungi, which comprises applying an effective amount of a compound of the formula I as claimed in claim 1 or 2 to these harmful fungi or the plants, surfaces or substrates infected with them.

## Revendications (Revendications pour l'(les) Etat(s) contractant(s) suivant(s): AT, BE, CH, DE, FR, GB, IT, LI, NL, SE)

1. Dérivés de pyrimidine de formule I 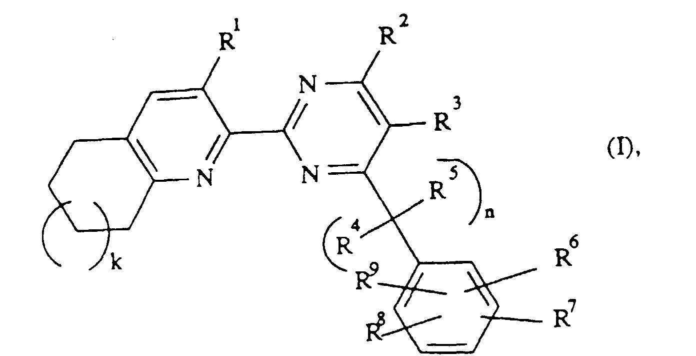 dans laquelle
R¹ = un atome d'hydrogène, un groupe alkyle en C₁-C₆, alcoxy en C₁-C₆, phénylalkyle en C₁-C₄, le fragment phényle pouvant être substitué jusqu'à trois fois par un atome d'halogène, un groupe alkyle en C₁-C₄, alcoxy en C₁-C₄, (alkyl en C₁-C₄)thio, halogénoalkyle en C₁-C₄, ou halogénoalcoxy en C₁-C₄,
R² = un atome d'hydrogène, un groupe hydroxy, (alcényl en C₂-C₄)-(alkyle en C₁-C₄), (alcynyl en C₂-C₄)-(alkyle en C₁-C₄), alkyle en C₁-C₆, (alcoxy en C₁-C₄)-(alkyle en C₁-C₄), alcényle en C₂-C₆, alcynyle en C₂-C₆, cycloalkyle en C₃-C₇, (cycloalkyl en C₃-C₇)-(alkyle en C₁-C₄), les deux derniers radicaux mentionnés dans le fragment cycloalkyle pouvant être substitués jusqu'à trois fois par un groupe alkyle en C₁-C₄, représente aussi un groupe phényle, phénoxyalkyle en C₁-C₄, phénylalkyle en C₁-C₄, les trois derniers radicaux mentionnés dans le fragment phényle pouvant être substitués jusqu'à trois fois par un atome d'halogène, un groupe alkyle en C₁-C₄, alcoxy en C₁-C₄, (alkyl en C₁-C₄)thio, halogénoalkyle en C₁-C₄ ou halogénoalcoxy en C₁-C₄, représente aussi alcoxy en C₁-C₁₂, (alcényl en C₂-C₆)-(alcoxy en C₁-C₄), (alcynyl en C₂-C₆)-(alcoxy en C₁-C₄), cycloalcoxy en C₃-C₇, (alcoxy en C₁-C₆)-(alcoxy en C₁-C₆), hydroxyalcoxy en C₁-C₆, halogénoalcoxy en C₁-C₆, (alkyl en C₁-C₆)thio, (alcényl en C₂-C₆)-(alkyl en C₁-C₄)thio, (alcynyl en C₂-C₆)-(alkyl en C₁-C₄)thio, (cycloalkyl en C₃-C₇)thio, phénoxy, phénylthio, phénylalcoxy en C₁-C₆, phényl(alkyl en C₁-C₆)thio, phényl(alcoxy en C₁-C₂)-(alcoxy en C₁-C₄), le cycle phényle des cinq derniers radicaux mentionnés pouvant être substitué jusqu'à trois fois par un atome d'halogéne, un groupe alkyle en C₁-C₄, alcoxy en C₁-C₄, (alkyl en C₁-C₄)thio, halogénoalkyle en C₁-C₄ ou halogénoalcoxy en C₁-C₄, ainsi qu'une fois par un groupe nitro ou cyano,
R³ = un atome d'hydrogène, un groupe alkyle en C₁-C₆, halogénoalkyle en C₁-C₄, alcoxy en C₁-C₄, (alkyl en C₁-C₄)thio, un atome d'halogène, un groupe phényle, phénylalkyle en C₁-C₄, le radical phényle des trois derniers radicaux mentionnés pouvant être substitué jusqu'à trois fois par un atome d'halogène, un groupe alkyle en C₁-C₄, alcoxy en C₁-C₄, (alkyl en C₁-C₄)thio, halogénoalkyle en C₁-C₄ ou halogénoalcoxy en C₁-C₄, ainsi qu'une fois par un groupe nitro ou cyano,
R⁴, R⁵ = indépendamment l'un de l'autre, ou un atome d'hydrogène, un groupe alkyle en C₁-C₆, ou
R³ avec R⁴ ou R⁵ représentant un fragment d'un cycle à 1 chaînons à insaturation maximale , avec 1 = 3 à 8,
R⁶, R⁷, R⁸, R⁹ = indépendamment les uns des autres, un atome d'hydrogène, un atome d'halogène, un groupe nitro, cyano, un groupe alkyle en C₁-C₆, alcoxy en C₁-C₆, (alkyl en C₁-C₆)thio, halogénoalkyle en C₁-C₆, halogénoalcoxy en C₁-C₆, aryle, aryloxy, les deux derniers radicaux mentionnés dans le radical aryle pouvant être substitués jusqu'à trois fois par un atome d'halogène, un groupe alkyle en C₁-C₄, alcoxy en C₁-C₄, halogénoalkyle en C₁-C₄ ou halogénoalcoxy en C₁-C₄,
deux des radicaux R⁶ à R⁹ représentant éventuellement un constituant d'un cycle insaturé ou saturé à m chaînons, avec m = 5 ou 6,
k = 0, 1 ou 2,
n = 1 ou 2, et
halogéno = substitué une ou plusieurs fois par un atome d'halogène,
ainsi que leurs sels d'addition d'acide.

2. Composés de formule I selon la revendication 1, caractérisés en ce que
R¹ = un atome d'hydrogène, alkyle en C1-C4, alcoxy en C₁-C₆,
R² = un atome d'hydrogène, un groupe hydroxy, alkyle en C₁-C₄, (alcényl en C₂-C₄)-(alkyle en C₁-C₂), (alcynyl en C₂-C₄)-(alkyle en C₁-C₂), (alcoxy en C₁-C₄)-(alkyle en C₁-C₄), alcényle en C₂-C₄, alcynyle en C₂-C₄, cycloalkyle en C₃-C₆, (cycloalkyl en C₃-C₆)-(alkyle en C₁-C₂), les deux derniers radicaux mentionnés du fragment cycloalkyle pouvant être substitués jusqu'à trois fois par un groupe alkyle en C₁-C₄, représente aussi phényle, phénoxyalkyle en C₁-C₂, phénylalkyle en C₁-C₂, les trois derniers radicaux mentionnés dans le fragment phényle pouvant être substitués jusqu'à trois fois par un atome d'halogène, un groupe alkyle en C₁-C₄, alcoxy en C₁-C₄, (alkyl en C₁-C₄)thio halogénoalkyle en C₁-C₄ ou halogénoalcoxy en C₁-C₄, un groupe alcoxy en C₁-C₁₂, (alcényl en C₂-C₄)-(alcoxy en C₁-C₂), (alcynyle en C₂-C₄)-(alcoxy en C₁-C₂), cycloalcoxy en C₃-C₆, (alcoxy en C₁-C₄)-(alcoxy en C₁-C₄), hydroxyalcoxy en C₁-C₄, halogénoalcoxy en C₁-C₆, (alkyl en C₁-C₆)thio, (alcényl en C₂-C₆)-(alkyl en C₁-C₂)thio, (alcynyl en C₂-C₄)-(alkyl en C₁-C₂)thio, (cycloalkyl en C₃-C₆)thio, phénoxy, phénylthio, phénylalcoxy en C₁-C₂, phényl(alkyl en C₁-C₂)thio, phényl(alcoxy en C₁-C₂)-(alcoxy en C₁-C₄), le cycle phényle dans les quatre derniers radicaux mentionnés pouvant être substitué jusqu'à trois fois par un atome d'halogéne, un groupe alkyle en C₁-C₄, alcoxy en C₁-C₄, (alkyl en C₁-C₄)thio, halogénoalkyle en C₁-C₄ ou halogénoalcoxy en C₁-C₄,
R³ = un atome d'hydrogène, un groupe alkyle en C₁-C₄, halogénoalkyle en C₁-C₄, alcoxy en C₁-C₄, (alkyl en C₁-C₄)thio, un atome d'halogène, un groupe phényle, phénylalkyle en C₁-C₂,
R⁴, R⁵ = indépendamment l'un de l'autre, un atome d'hydrogène, ou un groupe alkyle en C₁-C₂, ou
R³ avec R⁴ ou R⁵ représentant un constituant d'un cycle à 1 chaînons à insaturation maximale, avec 1 = 5 ou 6,
R⁶, R⁷, R⁸, R⁹ = indépendamment les uns des autres, un atome d'hydrogène, un atome d'halogène, un groupe nitro, cyano, un groupe alkyle en C₁-C₄, alcoxy en C₁-C₄, halogénoalkyle en C₁-C₄, halogénoalcoxy en C₁-C₄, phényle, phényloxy, les deux derniers radicaux mentionnés dans le radical phényle pouvant être substitués jusqu'à trois fois par un atome d'halogène, un groupe alkyle en C₁-C₄, alcoxy en C₁-C₄, halogénoalkyle en C₁-C₄, ou halogénoalcoxy en C₁-C₄,
deux des radicaux R⁶ à R⁹ représentent éventuellement un constituant d'un cycle insaturé ou saturé à m chaînons, avec m = 5 ou 6,
k = 0, 1 ou 2
n = 1 ou 2, et
halogéno = substitué par un ou plusieurs atomes d'halogène,
ainsi que leurs sels d'addition d'acide.

3. Procédé pour la préparation de composés de formule I selon la revendication 2, caractérisé en ce que
a) pour des composés avec R² = atome d'hydrogène, on déshalogéné par déshalogénation réductrice une halogénopyrimidine de formule I correspondante, avec R² = atome d'halogène et tous les autres substituants ayant les significations indiquées, ou
b) pour des composés de formule I, dans lesquels R² représente un radical organique se terminant par le suffixe "oxy" ou "thio" défini pour R² dans la revendication 1, on fait réagir une halogénopyrimidine correspondante de formule I avec R² = atome d'halogène, avec un composé de métal alcalin de formule II
R² - Y (II)
dans laquelle R² possède la signification indiquée ci-dessus et Y représente un métal alcalin, ou
c) pour un composé de formule I, dans laquelle R² représente un radical organique ne se terminant pas par le suffixe "oxy" ou "thio" défini pour R² dans la revendication 1, on fait réagir une halogénopyrimidine de formule I correspondante avec R² = un atome d'halogène, avec un composé de Grignard de formule III
R² - MgX (III),
dans laquelle R² possède la signification indiquée ci-dessus et X signifie halogène, en présence de complexes de nickel-phosphine.

4. Agents fongicides caractérisés en ce qu'ils contiennent une quantité efficace d'un composé de formule I selon la revendication 1 ou 2.

5. Utilisation de composés de formule I selon la revendication 1 ou 2 pour la lutte contre des champignons nuisibles.

6. Procédé de lutte contre des champignons nuisibles caractérisé en ce qu'on applique sur ceux-ci ou sur les végétaux, surfaces ou substrats attaqués par eux, une quantité efficace d'un composé de formule I selon la revendication 1 ou 2.

## Revendications (Revendications pour l'(les) Etat(s) contractant(s) suivant(s): ES)

1. Procédé pour la préparation de dérivés de pyrimidine de formule I 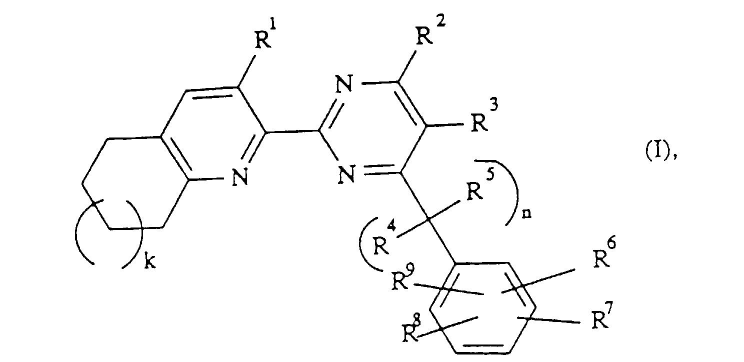 dans laquelle
R¹ = un atome d'hydrogène, un groupe alkyle en C₁-C₆, alcoxy en C₁-C₆, phénylalkyle en C₁-C₄, le fragment phényle pouvant être substitué jusqu'à trois fois par un atome d'halogène, un groupe alkyle en C₁-C₄, alcoxy en C₁-C₄, (alkyl en C₁-C₄) thio, : halogénoalkyle en C₁-C₄, ou halogénoalcoxy en C₁-C₄,
R² = un atome d'hydrogène, un groupe hydroxy, (alcényl en C₂-C₄)-(alkyle en C₁-C₄), (alcynyl en C₂-C₄)-(alkyle en C₁-C₄), alkyle en C₁-C₆, (alcoxy en C₁-C₄)-(alkyle en C₁-C₄), alcényle en C₂-C₆, alcynyle en C₂-C₆, cycloalkyle en C₃-C₇, (cycloalkyl en C₃-C₇)-(alkyle en C₁-C₄), les deux derniers radicaux mentionnés dans le fragment cycloalkyle pouvant être substitués jusqu'à trois fois par un groupe alkyle en C₁-C₄, représente aussi un groupe phényle, phénoxyalkyle en C₁-C₄, phénylalkyle en C₁-C₄, les trois derniers radicaux mentionnés dans le fragment phényle pouvant être substitués jusqu'à trois fois par un atome d'halogène, un groupe alkyle en C₁-C₄, alcoxy en C₁-C₄, (alkyl en C₁-C₄)thio, halogénoalkyle en C₁-C₄ ou halogénoalcoxy en C₁-C₄, représente aussi alcoxy en C₁-C₁₂, (alcényle en C₂-C₆)-(alcoxy en C₁-C₄), (alcynyl en C₂-C₆)-(alcoxy en C₁-C₄), cycloalcoxy en C₃-C₇, (alcoxy en C₁-C₆)-(alcoxy en C₁-C₆), hydroxyalcoxy en C₁-C₆, halogénoalcoxy en C₁-C₆, (alkyl en C₁-C₆)thio, (alcényl en C₂-C₆)-(alkyl en C₁-C₄)thio, (alcynyl en C₂-C₆)-(alkyl en C₁-C₄)thio, (cycloalkyl en C₃-C₇)thio, phénoxy, phénylthio, phénylalcoxy en C₁-C₆, phényl(alkyl en C₁-C₆)thio, phényl(alcoxy en C₁-C₂)-(alcoxy en C₁-C₄), le cycle phényle des cinq derniers radicaux mentionnés pouvant être substitué jusqu'à trois fois par un atome d'halogéne, un groupe alkyle en C₁-C₄, alcoxy en C₁-C₄, (alkyl en C₁-C₄)thio, halogénoalkyle en C₁-C₄ ou halogénoalcoxy en C₁-C₄, ainsi qu'une fois par un groupe nitro ou cyano,
R³ = un atome d'hydrogène, un groupe alkyle en C₁-C₆, halogénoalkyle en C₁-C₄, alcoxy en C₁-C₄, (alkyl en C₁-C₄)thio, un atome d'halogène, un groupe phényle, phénylalkyle en C₁-C₄, le radical phényle des trois derniers radicaux mentionnés pouvant être substitué jusqu'à trois fois par un atome d'halogène, un groupe alkyle en C₁-C₄, alcoxy en C₁-C₄, (alkyl en C₁-C₄)thio, halogénoalkyle en C₁-C₄ ou halogénoalcoxy en C₁-C₄, ainsi qu'une fois par un groupe nitro ou cyano,
R⁴, R⁵ = indépendamment l'un de l'autre, ou un atome d'hydrogène, un groupe alkyle en C₁-C₆, ou
R³ avec R⁴ ou R⁵ représentant un fragment d'un cycle à l chaînons à insaturation maximale , avec l = 3 à 8,
R⁶, R⁷, R⁸, R⁹ = indépendamment les uns des autres, un atome d'hydrogène, un atome d'halogène, un groupe nitro, cyano, un groupe alkyle en C₁-C₆, alcoxy en C₁-C₆, (alkyl en C₁-C₆)thio, halogénoalkyle en C₁-C₆, halogénoalcoxy en C₁-C₆, aryle, aryloxy, les deux derniers radicaux mentionnés dans le radical aryle pouvant être substitués jusqu'à trois fois par un atome d'halogène, un groupe alkyle en C₁-C_{4,} alcoxy en C₁-C₄, halogénoalkyle en C₁-C₄ ou halogénoalcoxy en C₁-C₄,
deux des radicaux R⁶ à R⁹ représentant éventuellement un constituant d'un cycle insaturé ou saturé à m chaînons, avec m = 5 ou 6,
k = 0, 1 ou 2,
n = 1 ou 2, et
halogéno = substitué une ou plusieurs fois par un atome d'halogène,
ainsi que leurs sels d'addition d'acide caractérisé en ce que
a) pour des composés avec R² = atome d'hydrogène, on déshalogéné par déshalogénation réductrice une halogénopyrimidine de formule I correspondante, avec R² = atome d'halogène et tous les autres substituants ayant les significations indiquées, ou
b) pour des composés de formule I, dans lesquels R² représente un radical organique se terminant par le suffixe "oxy" ou "thio" défini pour R^{2'} dans la revendication 1, on fait réagir une halogénopyrimidine correspondante de formule I avec R² = atome d'halogène, avec un composé de métal alcalin de formule II
R² - Y (II)
dans laquelle R² possède la signification indiquée ci-dessus et Y représente un métal alcalin, ou
c) pour un composé de formule I, dans laquelle R² représente un radical organique ne se terminant pas par le suffixe "oxy" ou "thio" défini pour R² dans la revendication 1, on fait réagir une halogénopyrimidine de formule I correspondante avec R² = un atome d'halogène, avec un composé de Grignard de formule III
R² - MgX (III),
dans laquelle R² possède la signification indiquée ci-dessus et X signifie halogène, en présence de complexes de nickel-phosphine.

2. Procédé selon la revendication 1, caractérisés en ce que
R¹ = un atome d'hydrogène, alkyle en C₁-C₄, alcoxy en C₁-C₆,
R² = un atome d'hydrogène, un groupe hydroxy, alkyle en C₁-C₄, (alcényl en C₂-C₄)-(alkyle en C₁-C₂), (alcynyl en C₂-C₄)-(alkyle en C₁-C₂), (alcoxy en C₁-C₄)-(alkyle en C₁-C₄), alcényle en C₂-C₄, alcynyle en C₂-C₄, cycloalkyle en C₃-C₆, (cycloalkyl en C₃-C₆)-(alkyle en C₁-C₂), les deux derniers radicaux mentionnés du fragment cycloalkyle pouvant être substitués jusqu'à trois fois par un groupe alkyle en C₁-C₄, représente aussi phényle, phénoxyalkyle en C₁-C₂, phénylalkyle en C₁-C₂, les trois derniers radicaux mentionnés dans le fragment phényle pouvant être substitués jusqu'à trois fois par un atome d'halogène, un groupe alkyle en C₁-C₄, alcoxy en C₁-C₄, (alkyl en C₁-C₄)thio halogénoalkyle en C₁-C₄ ou halogénoalcoxy en C₁-C₄, un groupe alcoxy en C₁-C₁₂, (alcényl en C₂-C₄)-(alcoxy en C₁-C₂), (alcynyle en C₂-C₄)-(alcoxy en C₁-C₂), cycloalcoxy en C₃-C₆, (alcoxy en C₁-C₄)-(alcoxy en C₁-C₄), hydroxyalcoxy en C₁-C₄, halogénoalcoxy en C₁-C₆, (alkyl en C₁-C₆)thio, (alcényl en C₂-C₆)-(alkyl en C₁-C₂)thio, (alcynyl en C₂-C₄)-(alkyl en C₁-C₂)thio, (cycloalkyl en C₃-C₆)thio, phénoxy, phénylthio, phénylalcoxy en C₁-C₂, phényl(alkyl en C₁-C₂)thio, phényl(alcoxy en C₁-C₂)-(alcoxy en C₁-C₄), le cycle phényle dans les quatre derniers radicaux mentionnés pouvant être substitué jusqu'à trois fois par un atome d'halogéne, un groupe alkyle en C₁-C₄, alcoxy en C₁-C₄, (alkyl en C₁-C₄)thio, halogénoalkyle en C₁-C₄ ou halogénoalcoxy en C₁-C₄,
R³ = un atome d'hydrogène, un groupe alkyle en C₁-C₄, halogénoalkyle en C₁-C₄, alcoxy en C₁-C₄, (alkyl en C₁-C4)thio, un atome d'halogène, un groupe phényle, phénylalkyle en C₁-C₂,
R⁴, R⁵ = indépendamment l'un de l'autre, un atome d'hydrogène, ou un groupe alkyle en C₁-C₂, ou
R³ avec R⁴ ou R⁵ représentant un constituant d'un cycle à 1 chaînons à insaturation maximale, avec 1 = 5 ou 6,
R⁶, R⁷, R⁸, R⁹ = indépendamment les uns des autres, un atome d'hydrogène, un atome d'halogène, un groupe nitro, cyano, un groupe alkyle en C₁-C₄, alcoxy en C₁-C₄, halogénoalkyle en C₁-C₄, halogénoalcoxy en C₁-C₄, phényle, phényloxy, les deux derniers radicaux mentionnés dans le radical phényle pouvant être substitués jusqu'à trois fois par un atome d'halogène, un groupe alkyle en C₁-C₄, alcoxy en C₁-C₄, halogénoalkyle en C₁-C₄, ou halogénoalcoxy en C₁-C₄,
deux des radicaux R⁶ à R⁹ représentent éventuellement un constituant d'un cycle insaturé ou saturé à m chaînons, avec m = 5 ou 6,
k = 0, 1 ou 2
n = 1 ou 2, et
halogéno = substitué par un ou plusieurs atomes d'halogène,
ainsi que leurs sels d'addition d'acide.

3. Agents fongicides, caractérisés en ce qu'ils contiennent une quantité efficace d'un composé de formule I selon la revendication 1 ou 2.

4. Utilisation de composés de formule I selon la revendication 1 ou 2, pour la lutte contre des champignons nuisibles.

5. Procédé de lutte contre des champignons nuisibles, caractérisé en ce qu'on applique sur ceux-ci ou sur des végétaux, des surfaces ou des substrats attaqués par eux, une quantité efficace d'un composé de formule I selon la revendication 1 ou 2.
